# EUROPÄISCHE PATENTANMELDUNG

(11) **EP 0 927 761 A2**
(43) Veröffentlichungstag der Anmeldung: **07.07.1999**
(21) Anmeldenummer: 98123331.5
(22) Anmeldetag: 08.12.1998
(51) Int. Cl.: C12N 15/52, C12N 15/53, C12N 15/54, C12P 25/00, C12N 9/00, C12N 9/04, C12N 9/10, C12N 9/12

(54) **Gene der Purinbiosyntese aus Ashbya gossypii und deren Verwendung in der mikrobiellen Riboflavinsynthese**

(30) Priorität: 23.12.1997 DE 19757755
(71) Anmelder: BASF AKTIENGESELLSCHAFT, 67056 Ludwigshafen (DE)
(72) Erfinder: Pompejus, Markus Dr., 67165 Waldsee (DE); Seulberger, Harald Dr., 67141 Neuhofen (DE); Höffken, Hans Wolfgang Dr., 67069 Ludwigshafen (DE); Revuelta Doval, Jose Luis Prof.-Dr., 37001 Salamanca (ES); Jimenez, Alberto, 37006 Salamanca (ES); Santos Garcia, Maria Angeles Dr., 37009 Salamanca (ES)

(57) **Zusammenfassung**

Gene der Purinbiosynthese aus Ashbya gossypii und deren Verwendung in der mikrobiellen Riboflavinsynthese.

## Beschreibung

Die vorliegende Erfindung betrifft Gene der Purinbiosynthese aus Ashbya gossypii und deren Verwendung in der Riboflavinsynthese.

Vitamin B2, auch Riboflavin genannt, ist für Mensch und Tier essentiell. Bei Vitamin B2-Mangel treten Entzündungen der Mund- und Rachenschleimhäute, Juckreiz und Entzündungen in den Hautfalten und ähnliche Hautschäden, Bindehautentzündungen, verminderte Sehschärfe und Trübung der Hornhaut auf. Bei Säuglingen und Kindern können Wachstumsstillstand und Gewichtsabnahme eintreten. Vitamin B2 hat daher wirtschaftliche Bedeutung insbesondere als Vitaminzusatz bei Vitaminmangel und als Futtermittelzusatz. Daneben wird es als Lebensmittelfarbstoff, beispielsweise in Mayonnaise, Eiscreme, Pudding etc. eingesetzt.

Die Herstellung von Vitamin B2 erfolgt entweder chemisch oder mikrobiell (siehe z.B. Kurth et al. (1996) Riboflavin, in: Ullmann's Encyclopedia of industrial chemistry, VCH Weinheim). Bei den chemischen Herstellverfahren wird Riboflavin in der Regel in mehrstufigen Prozessen als reines Endprodukt gewonnen, wobei relativ kostspielige Ausgangsprodukte, wie z.B. D-Ribose, eingesetzt werden müssen. Eine Alternative zur chemischen Synthese von Riboflavin ist die Herstelltung dieses Stoffes durch Mikroorganismen. Als Ausgangsstoffe dienen dabei nachwachsende Rohstoffe, wie Zucker oder pflanzliche Öle. Die Herstellung von Riboflavin durch Fermentation von Pilzen wie Eremothecium ashbyii oder Ashbya gossypii ist bekannt (The Merck Index, Windholz et al., eds. Merck & Co., Seite 1183, 1983), aber auch Hefen, wie z.B. Candida, Pichia und Saccharomyces oder Bakterien, wie z.B. Bacillus, Clostridien oder Corynebakterien sind als Riboflavin-Produzenten beschrieben.

In der EP 405370 sind Riboflavin-überproduzierende Bakerienstämme beschrieben, die durch Transformation der Riboflavin-Biosynthese-Gene aus Bacillus subtilis erhalten wurden. Diese dort beschriebenen Gene und andere, an der Vitamin B2-Biosynthese beteiligten Gene aus Prokaryonten sind für ein rekombinantes Riboflavin-Herstellverfahren mit Eukaryonten, wie z.B. Saccharomyces cerevisiae oder Ashbya gossypii ungeeignet.

In der DE 44 20 785 sind sechs Riboflavin-Biosynthesegene aus Ashbya gossypii beschrieben, sowie Mikroorganismen, die mit diesen Genen transformiert wurden und die Verwendung solcher Mikroorganismen zur Riboflavinsynthese.

Mit diesen Verfahren ist es möglich, Produktionsstämme für die mikrobielle Riboflavinsynthese zu erzeugen. Diese Produktionsstämme besitzen jedoch häufig Stoffwechsellimitierungen, die durch die insertierten Biosynthesegene nicht beseitigt werden können oder manchmal erst dadurch entstehen. Solche Produktionsstämmen können manchmal nicht genügend Substrat zur Sättigung mancher Biosyntheseschritte liefern, so daß die Biosynthesekapazität mancher Stoffwechselabschnitte gar nicht voll ausgeschöpft werden kann.

Daher ist es wünschenswert, weitere Teilbereiche von Stoffwechselwegen zu verstärken, dadurch Stoffwechselengpässe zu beseitigen und dadurch dann die für die mikrobielle Riboflavinsynthese eingesetzten Mikroorganismen (Produktionsstämme) bezüglich ihrer Fähigkeit zur Riboflavinsynthese weiter zu optimieren. Es ist anzustreben, die zu verstärkenden Teilbereiche des komplexen Stoffwechsels zu identifizieren und diese auf geeignete Weise zu verstärken.

Die Erfindung betrifft neue Proteine der Purinbiosynthese, deren Gene und deren Verwendung für die mikrobielle Riboflavinsynthese.

Der Purinstoffwechsel (für eine Übersicht siehe z.B. Voet, D. und Voet, J.G., 1994, Biochemie, VCH Weinheim, Seite 743-771; Zalkin, H. und Dixon, J.E., 1992, De novo purine nucleotide biosynthesis, in: Progress in nucleic acid research and molecular biology, Vol. 42, Seite 259-287, Academic Press) ist ein für alle Lebewesen essentieller Teil des Stoffwechsels. Fehlerhafter Purinstoffwechsel kann beim Menschen zu schweren Krankheiten führen (z.B. Gicht). Der Purinstoffwechsel ist zudem ein wichtiges target für die Therapie von Tumorerkrankungen und Virusinfektionen. Zahllose Publikationen sind erschienen, die Substanzen für diese Indikationen beschreiben die im Purinstoffwechsel eingreifen (als Übersicht z.B. Christopherson, R.I. und Lyons, S.D., 1990, Potent inhibitors of de novo pyrimidine and purine biosynthesis as chemotherapeutic agents, Med. Res. Reviews 10, Seite 505-548).

Untersuchungen der in Purinstoffwechsel beteiligten Enzyme (Smith, J.L., Enzymes in nucleotide synthesis, 1995, Curr. Opinion Struct. Biol. 5, 752-757) zielen darauf ab, neue immunsuppressiv, anti-parasitär oder anti-proliferierend wirkende Medikamente zu entwickeln (Biochem. Soc. Transact. 23, Seite 877-902, 1995).

Bei diesen Medikamenten handelt es sich dann üblicherweise um natürlich nicht vorkommende Purine, Pyrimidine oder davon abgeleiteter Verbindungen.

Gegenstand der Erfindung ist ein Protein mit der in SEQ ID NO:2 dargestellten Polypeptidseqenz oder einer aus SEQ ID NO:2 durch Substitution, Insertion oder Deletion von bis zu 15% der Aminosäuren erhältlichen Polypeptidsequenz und der enzymatischen Aktivität einer Phosphoribosylpyrophosphat-Synthetase.

Die in SEQ ID NO:2 dargestellte Sequenz ist das aus Ashbya gossypii erhaltene Genprodukt des KPR1 Gens (SEQ ID NO:1).

Ein weiterer Gegenstand der Erfindung ist ein Protein mit der in SEQ ID NO:5 dargestellten Polypeptidseqenz oder einer aus SEQ ID NO:5 durch Substitution, Insertion oder Deletion von bis zu 10% der Aminosäuren erhältlichen Polypeptidsequenz und der enzymatischen Aktivität einer Glutamin-Phosphoribosylpyrophosphat-Amidotransferase.

Die in SEQ ID NO:5 dargestellte Sequenz ist das aus Ashbya gossypii erhaltene Genprodukt des ADE4 Gens (SEQ ID NO:3).

Ein weiterer Gegenstand der Erfindung ist ein Protein mit der in SEQ ID NO:8 dargestellten Polypeptidseqenz oder einer aus SEQ ID NO:8 durch Substitution, Insertion oder Deletion von bis zu 20% der Aminosäuren erhältlichen Polypeptidsequenz und der enzymatischen Aktivität einer IMP-Dehydrogenase.

Die in SEQ ID NO:8 und 9 dargestellte Sequenz ist das aus Ashbya gossypii erhaltene Genprodukt des GUA1 Gens (SEQ ID NO:7).

Ein weiterer Gegenstand der Erfindung ist ein Protein mit der in SEQ ID NO:11 dargestellten Polypeptidseqenz oder einer aus SEQ ID NO:11 durch Substitution, Insertion oder Deletion von bis zu 10% der Aminosäuren erhältlichen Polypeptidsequenz und der enzymatischen Aktivität einer GMP-Synthetase.

Die in SEQ ID NO:11 dargestellte Sequenz ist das aus Ashbya gossypii erhaltene Genprodukt des GUA2 Gens (SEQ ID NO:10).

Ein weiterer Gegenstand der Erfindung ist ein Protein mit der in SEQ ID NO:13 dargestellten Polypeptidseqenz oder einer aus SEQ ID NO:13 durch Substitution, Insertion oder Deletion von bis zu 10% der Aminosäuren erhältlichen Polypeptidsequenz und der enzymatischen Aktivität einer Phosphoribosylpyrophosphat-Synthetase.

Die in SEQ ID NO:13 dargestellte Sequenz ist das aus Ashbya gossypii erhaltene Genprodukt des KPR2 Gens (SEQ ID NO:12).

Diese genannten Genprodukte können durch übliche Verfahren der Gentechnologie wie ortsgerichtete Mutagenese so verändert werden, daß bestimmte Aminosäuren ausgetauscht, zusätzlich eingeführt oder entfernt werden. Üblicherweise (aber nicht ausschließlich) werden Aminosäurereste durch solche ähnlicher Raumerfüllung, Ladung oder Hydrophilie/Hydrophobie ausgetauscht um die enzymatischen Eigenschaften der Genprodukte nicht zu verlieren. Insbesondere im aktiven Zentrum führen Veränderungen der Aminosäuresequenz oft zu drastischer Veränderung der enzymatischen Aktivitäten. An anderen, weniger essentiellen Stellen werden jedoch Veränderungen der Aminosäuresequenz häufig toleriert.

Bei den erfindungsgemäßen Proteinen können
1. im Fall des Genproduktes des AgKPR1-Gens, bis zu 15, bevorzugt bis zu 10 und besonders bevorzugt bis zu 5% der Aminosäuren gegenüber der im Sequenzprotokoll dargestellten Sequenzen verändert sein;
2. im Fall des Genproduktes des AgADE4-Gens, bis zu 10 und besonders bevorzugt bis zu 5% der Aminosäuren gegenüber der im Sequenzprotokoll dargestellten Sequenzen verändert sein;
3. im Fall des Genproduktes des AgGUA1-Gens, bis zu 20, bevorzugt bis zu 15, besonders bevorzugt bis zu 10 und insbesondere bevorzugt bis zu 5% der Aminosäuren gegenüber der im Sequenzprotokoll dargestellten Sequenzen verändert sein;
4. im Fall des Genproduktes des AgGUA2-Gens, bis zu 10 und besonders bevorzugt bis zu 5% der Aminosäuren gegenüber der im Sequenzprotokoll dargestellten Sequenzen verändert sein;
5. im Fall des Genproduktes des AgKPR2 Gens bis zu 10 %, bevorzugt bis zu 7 % und besonders bevorzugt bis zu 5 % der Aminosäuren gegenüber der im Sequenzprotokoll dargestellten Sequenzen verändert sein.

Bevorzugt sind solche Proteine, die zwar noch die jeweilige enzymatische Aktivität besitzen, aber in ihrer Regulation verändert worden sind. Viele dieser Enzyme unterliegen einer starken Aktivitätskontrolle durch Zwischen- und Endprodukte (feedback-Inhibition). Dies führt dazu, daß die Enzyme, sobald genügend Endprodukt vorhanden ist, in ihrer Aktivität gedrosselt werden.

Diese im physiologischen Zustand ökonomische Regelung führt bei Produktionsstämmen jedoch häufig dazu, daß die Produktivität nicht über eine gewisse Grenze hinaus gesteigert werden kann. Durch Beseitigung einer solchen feedback-Inhibition erreicht man, daß die Enzyme ungeachtet der Endproduktkonzentration ihre Aktivität beibehalten und dadurch Stoffwechselengpässe umgangen werden. Dies führt letztlich zu einer deutlichen Steigerung der Riboflavinbiosynthese.

Bevorzugte erfindungsgemäße Proteine sind solche, die nicht mehr durch Folgeprodukte von Stoffwechselwegen (die von Produkten der Enzyme ausgehen) gehemmt werden. Besonders bevorzugte erfindungsgemäße Proteine sind solche, die nicht mehr durch Zwischenprodukte der Purinbiosynthese, insbesondere durch Purinbasen, Purinnukleoside, Purinnukleotid-5'-Monophosphate oder Purinnukleotid-5'-Diphosphate oder Purinnukleotid-5'-Triphosphate gehemmt werden. Insbesondere bevorzugte erfindungsgemäße Proteine sind solche, mit nachfolgenden Veränderungen der Aminosäuresequenz und alle Kombinationen von Aminosäuresequenz-Veränderungen, die diese nachfolgenden Veränderungen enthalten.

Veränderungen der Aminosäuresequenz am AgKPR1 Genprodukt:
Lysin an Position 7 ausgetauscht gegen Valin Aspartat an Position 52 ausgetauscht gegen Histidin Leucin an Position 131 ausgetauscht gegen Isoleucin Aspartat an Position 186 ausgetauscht gegen Histidin Alanin an Position 193 ausgetauscht gegen Valin Histidin an Position 196 ausgetauscht gegen Glutamin

Veränderungen der Aminosäuresequenz am AgADE4 Genprodukt:
Aspartat an Position 310 ausgetauscht gegen Valin Lysin an Position 333 ausgetauscht gegen Alanin Alanin an Position 417 ausgetauscht gegen Tryptophan

Die folgenden Beispiele beschreiben die Herstellung der erfindungsgemäßen Proteine und Nukleinsäuren sowie deren Verwendung zur Herstellung von Mikroorganismen mit gesteigerter Riboflavinsynthese.

### Beispiel 1:

### Herstellung einer genomischen Genbank aus Ashbya gossypii ATCC10895

Genomische DNA aus Ashbya gossypii ATCC10895 kann nach üblichen Verfahren präpariert werden, z.B. wie beschrieben in EP9703208. Die genomische Genbank, ausgehend von dieser DNA, kann nach üblichen Methoden (z.B. Sambrook, J. et al. (1989) Molecular cloning: a laboratory manual, Cold Spring Harbor Laboratory Press oder Ausubel, F.M. et al. (1994) Current protocols in molecular biology, John Wiley and sons) in beliebigen Plasmiden oder Cosmiden, wie z.B. SuperCos1 (Stratagene, La Jolla, USA) erstellt werden.

### Beispiel 2:

### Klonierung des Gens für PRPP-Synthetase aus Ashbya gossypii ATCC10895 (AgKPR1)

Die Klonierung des Gens für PRPP-Synthetase aus Ashbya gossypii (AgKPR1) kann über zwei Schritte verlaufen. Im ersten Schritt kann mit folgenden Oligonukleotiden einen definierten Bereich des KPR1 Gens aus genomischer DNA von Ashbya gossypii über PCR amplifizieren:
KPR5: 5'- GATGCTAGAGACCGCGGGGTGCAAC -3'
KPR3: 5'- TGTCCGCCATGTCGTCTACAATAATA -3'

Die PCR kann nach üblicher Methode durchgeführt werden. Das resultierende 330 bp DNA Fragment kann mit üblichen Methoden in den Vektor pGEMT (Promega, Madison, USA) kloniert und sequenziert werden.

Mit dieser Nukleotidsequenz als Sonde kann mit üblichen Methoden eine genomische Cosmid-Genbank gescreent werden. Von einem Cosmid, das ein Signal mit dieser Sonde ergibt, kann dann ein 1911 bp PstI-HindIII Fragment in den Vektor pBluescript SK+ (Stratagene, La Jolla, USA) subkloniert werden. Auf diesem Fragment liegen das KPR1 Gen und unvollständige ORFs, die Homologie zeigen zu den UBC6 und UBP9 Genen aus Saccharomyces cerevisiae.

Die PRPP-Synthetase KPR2 und die putative PRPP-Synthetase KPR4 aus Saccharomyces cerevisiae sind die Enzyme, die der PRPP-Synthetase aus Ashbya gossypii mit Ähnlichkeiten von 80,2% bzw. 79,6% am verwandtesten sind. Die KPR2 und KPR4 Gene aus Saccharomyces cerevisiae sind zu 67.6% bzw. 67.8% ähnlich zum KPR1 Gen aus Ashbya gossypii. Andere Enzyme bzw. Gene aus anderen Organismen sind deutlich verschiedener zum KPR1 Gen bzw. zur PRPP-Synthetase aus Ashbya gossypii.

Die Sequenzvergleiche können z.B. mit dem Clustal Algorithmus mit Hilfe der PAM250 Gewichtungstabelle oder dem Wilbur-Lipman DNA alignment Algorithmus (wie z.B. in dem Programmpaket MegAlign 3.06 der Firma DNAstar implementiert) durchgeführt werden. Mit dem beschriebenen Oligonukleotide-Paar ist es nicht möglich, die Gene für die verschiedenen PRPP-Synthetasen aus Saccharomyces cerevisiae zu amplifizieren.

Mit der Sonde kann man auch noch einen Klon aus der Genbank finden. Dieser zweite Klon zeigte ein Gen, das ebenfalls für eine PRPP Synthetase kodiert. Dieses Gen wird AgKPR2 genannt und ist deutlich verschieden zu AgKPR1. AgKPR2 zeigt auf Aminosäureebene 66 % Identität zu AgKPR1. Das AgKPR2 Gen (SEQ ID NO: 12) wurde mit allen Proteinen der Swissprot Datenbank verglichen. Die maximale Ähnlichkeit zeigt dieses Protein (88 % Identität bzw. 95 % Äknlichkeit) zum KPR3 Genprodukt aus Saccharomyces cerevisiae. Das Genprodukt des AgKPR1 Gens ist für den überwiegenden Teil der Aktivität der PRPP Synthetase bei Ashbya gossypii verantwortlich. Wenn man das AgKPR1 Gen von Ashbya gossypii disruptiert (analog zur Disruption anderer Ashbya Gene, wie in den Beschreibungen in den Beispielen 6-8), dann findet man deutlich verringerte Enzymaktivität: statt 22 U/mg Protein nur noch 3 U/mg Protein. Zur Analytik siehe Beispiel 13. In Beispiel 11, 13 und 15 werden Ausführungsbeispiele mit dem AgKPR1 Gen gezeigt, man kann solche Arbeiten aber auch mit AgKPR2 durchführen.

### Beispiel 3:

### Klonierung des Gens für Glutamin-PRPP-Amidotransferase aus Ashbya gossypii ATCC10895 (AgADE4)

Die Klonierung des Gens für Glutamin-PRPP-Amidotransferase aus Ashbya gossypii (AgADE4) kann über zwei Schritte verlaufen. Im ersten Schritt kann mit folgenden Oligonukleotiden einen definierten Bereich des AgADE4 Gens aus genomischer DNA von Ashbya gossypii über PCR amplifizieren:
ADE4A: 5'- ATATCTTGATGAAGACGTTCACCGT -3'
ADE4B: 5'- GATAATGACGGCTTGGCCGGGAAGA -3'

Die PCR kann nach üblicher Methode durchgeführt werden. Das resultierende 360 bp DNA Fragment kann mit üblichen Methoden in den Vektor pGEMT (Promega, Madison, USA) kloniert und dann sequenziert werden.

Mit dieser Sequenz als Sonde kann mit üblichen Methoden eine genomische Cosmid-Genbank gescreent werden. Von einem Cosmid, das ein Signal mit dieser Sonde ergibt, kann dann ein 5369 bp HindIII Fragment in den Vektor pBluescript SK+ (Stratagene, La Jolla, USA) subkloniert werden. Auf diesem Fragment liegen das AgADE4 Gen und das Gen für das Ashbya Homolog für den mitochondrialen ABC Transporter ATM1 aus Saccharomyces cerevisiae und ein weiterer offener Leseraster, dessen Funktion nicht bekannt ist.

Das AgADE4 Genprodukt (Glutamin-PRPP-Amidotransferase) zeigt die deutlichste Ähnlichkeit zu den ADE4 Genprodukten aus Saccharomyces cerevisiae und Saccharomyces kluyveri (81% bzw. 86.3%). Die entsprechenden Gene sind jedoch nur zu 68.8% bzw. 72% homolog. Die Ähnlichkeit zu anderen Glutamin-PRPP-Amidotransferasen ist deutlich geringer (z.B. nur 27.5% Ähnlichkeit zum entsprechenden Enzym aus Bacillus subtilis). Die Sequenzvergleiche kann man so durchführen, wie in Beispiel 2 beschrieben.

Es ist mit dem beschriebenen Paar an Oligonukleotiden nicht möglich, die ADE4 Gene aus Saccharomyces cerevisiae oder Saccharomyces kluyveri zu amplifizieren.

### Beispiel 4:

### Klonierung des Gens für Inosin-Monophosphat-Dehydrogenase aus Ashbya gossypii ATCC10895 (AgGUA1)

Die Klonierung des Gens für Inosin-Monophosphat-Dehydrogenase aus Ashbya gossypii (AgGUA1) kann über zwei Schritte verlaufen.

Im ersten Schritt kann man mit folgenden Oligonukleotiden einen definierten Bereich des AgGUA1 Gens aus genomischer DNA von Ashbya gossypii über PCR amplifizieren:
IMP5: 5'- GGCATCAACCTCGAGGAGGCGAACC -3'
IMP3: 5'- CAGACCGGCCTCGACCAGCATCGCC - 3'

Die PCR kann nach üblicher Methode durchgeführt werden. Das resultierende 230 bp DNA Fragment kann mit üblichen Methoden in den Vektor pGEMT (Promega, Madison, USA) kloniert und dann sequenziert werden.

Mit dieser Sequenz als Sonde kann mit üblichen Methoden eine genomische Cosmid-Genbank gescreent werden. Von einem Cosmid, das ein Signal mit dieser Sonde ergibt, kann ein 3616 bp ApaI Fragment in den Vektor pBluescript SK+ (Stratagene, La Jolla, USA) subkloniert werden. Die kodierende Region des AgGUA1 Gens Gens ist 1569 bp lang und ist unterbrochen von einem161 bp langen Intron. Die Intron Grenzen (5' splice site AGGTATGT und 3' splice site CAG) kann man durch Klonierung und Sequenzierung von AgGUA1cDNA verifizieren.

AgGUA1 ist das erste beschriebene Gen aus Ashbya gossypii mit einem Intron.

Das AgGUA1 Genprodukt (IMP-Dehydrogenase) zeigt die deutlichste Ähnlichkeit zu den 4 IMP-Dehydrogenasen aus Saccharomyces cerevisiae (Ähnlichkeiten zwischen 67% und 77.2%). Die Ähnlichkeit zu anderen IMP-Dehydrogenasen ist deutlich geringer Die Sequenzvergleiche kann man so durchführen, wie in Beispiel 2 beschrieben. Ashbya gossypii scheint nur ein Gen für dieses Enzym zu haben. Dies kann man durch southern blotting mit genomischer DNA von Ashbya gossypii mit Hilfe der oben genannter Sonde zeigen.

Das Gen aus Saccharomyces cerevisiae, das für die dem AgGUA1 Genprodukt ähnlichste IMP-Dehydrogenase kodiert (IMH3), hat eine Ähnlichkeit von 70.2% zum AgGUA1 Gen. Es ist mit dem beschriebenen Paar an Oligonukleotiden nicht möglich, dieses Gen aus Saccharomyces cerevisiae zu amplifizieren.

### Beispiel 5:

### Klonierung des Gens für Guanosin-Monophosphat-Svnthetase aus Ashbya gossypii ATCC10895 (AgGUA2)

Die Klonierung des Gens für Guanosin-Monophosphat-Synthetase aus Ashbya gossypii (AgGUA2) kann über zwei Schritte verlaufen. Im ersten Schritt kann man mit folgenden Oligonukleotiden einen definierten Bereich des AgGUA2 Gens aus genomischer DNA von Ashbya gossypii über PCR amplifizieren:
GUA2A: 5'- TGGACCGGGCGGTGTTCGAGTTGGG -3'
GUA2B: 5'- AGGCTGGATCCTGGCTGCCTCGCGC -3'

Die PCR Reaktion kann nach üblicher Methode durchgeführt werden. Das resultierende 750 bp DNA Fragment kann mit üblichen Methoden in den Vektor pBluescript SK+ (Stratagene, La Jolla, USA) kloniert und dann sequenziert werden.

Mit dieser Sequenz als Sonde kann mit üblichen Methoden eine genomische Cosmid-Genbank gescreent werden. Von einem Cosmid, das ein Signal mit dieser Sonde ergibt, kann dann ein 2697 bp ClaI-EcoRV Fragment in den Vektor pBluescript SK+ (Stratagene, La Jolla, USA) subkloniert werden.

Das AgGUA2 Genprodukt (GMP-Synthetase) zeigt die deutlichste Ähnlichkeit zu GMP-Synthetase aus Saccharomyces cerevisiae (Ähnlichkeiten 86.6%). Die Gene für die GMP-Synthetasen aus Saccharomyces cerevisiae und Ashbya gossypii sind zu 71.2% homolog. Die Ähnlichkeit des AgGUA2 Genproduktes zu anderen IMP-Dehydrogenasen ist deutlich geringer Die Sequenzvergleiche kann man so durchführen, wie in Beispiel 2 beschrieben.

Es ist mit dem beschriebenen Paar an Oligonukleotiden nicht möglich, das GMP-Synthetase Gen aus Saccharomyces cerevisiae zu amplifizieren.

### Beispiel 6:

### Disruption des AgADE4 Gens von Ashbya gossypii ATCC10895

Unter Disruption eines Genes versteht man die Zerstörung der Funktionalität einer genomischen Kopie des Gens entweder durch (a) Entfernen eines Teiles der Gensequenz, oder durch (b) der Unterbrechung des Gens durch Einfügung eines Stückes Fremd-DNA in das Gen oder durch (c) Ersatz eines Teil des Gens durch Fremd-DNA. Die verwendete Fremd-DNA ist beliebig, bevorzugt aber ein Gen, das Resistenz gegen eine beliebige Chemikalie bewirkt. Zur Disruption von Genen können beliebige Resistenzgene verwendet werden.

Zur Disruption des AgADE4-Gens von Ashbya gossypii ATCC10895 kann man ein Gen verwenden, das Resistenz gegen G418 vermittelt. Es kann sich dabei um das Kanamycin-Resistenzgen aus TN903, unter Kontrolle des TEF-Promotors von Ashbya gossypii (siehe z.B. Yeast 10, S.1793-1808, 1994, WO9200379) handeln. Das Gen ist 5' und 3' von mehreren Schnittstellen für Restriktionsendonukleasen flankiert, so daß eine Kassette aufgebaut wurde, die beliebige Konstruktionen von Gen-Disruptionen mit üblichen Methoden der in vitro Manipulation von DNA ermöglichen.

Das interne HincII Fragment von AgADE4 (zwischen den Positionen 2366 und 2924) kann durch eine wie oben skizzierte Resistenzkassette ersetzt werden. Das erhaltene Konstrukt erhält den Namen ade4::G418.

Das erhaltene Plasmid kann man in E.coli vermehren. Das BamHI / BglII- Fragment des Konstruktes ade4::G418 kann präpariert, über Agarosegel-Elektrophorese und nachfolgende Elution der DNA aus dem Gel (siehe Proc. Natl. Acad. Sci. USA 76, 615-619, 1979) aufgereinigt und zur Transformation von Ashbya gossypii eingesetzt werden.

Ashbya gossypii kann durch Protoplastentransformation (Gene 109, 99-105, 1991), bevorzugt aber durch Elektroporation (BioRad Gene Pulser, Bedingungen: Küvetten mit Spaltbreite 0,4 mm, 1500V, 25µF, 100Ω) transformiert werden. Die Selektion transformierter Zellen erfolgt auf G418-haltigem Festmedium.

Erhaltene G418-resistente Klone können mit üblichen Methoden der PCR und Southern-Blot Analyse daraufhin untersucht werden, ob die genomische Kopie des AgADE4 Gens tatsächlich zerstört ist. Klone, deren AgADE4 Gen zerstört ist, sind Purin- auxotroph.

### Beispiel 7:

### Disruption des AgGUA1 Gens von Ashbya gossypii ATCC10895

Zur prinzipiellen Beschreibung der Disruption von Genen, der Verwendung einer Resistenzkassette und der Transformation von Ashbya gossypii siehe Beispiel 6.

Das interne XhoI / KpnI Fragment von AgGUA1 (zwischen den Positionen 1620 und 2061) kann durch eine wie oben skizzierte Resistenzkassette ersetzt werden. Das erhaltene Konstrukt erhält den Namen gua1::G418.

Das erhaltene Plasmid kann in E.coli vermehrt werden. Das XbaI / BamHI - Fragment des Konstruktes gua1::G418 kann präpariert, über Agarosegel-Elektrophorese und nachfolgende Elution der DNA aus dem Gel aufgereinigt und zur Transformation von Ashbya gossypii eingesetzt werden.

Erhaltene G418-resistente Klone können mit üblichen Methoden der PCR und Southern-Blot Analyse daraufhin untersucht werden, ob die genomische Kopie des AgGUA1 Gens tatsächlich zerstört ist. Klone, deren AgGUA1 Gen zerstört ist, sind Guanin- auxotroph.

### Beispiel 8:

### Disruption des AgGUA2 Gens von Ashbya gossypii ATCC10895

Zur prinzipiellen Beschreibung der Disruption von Genen, der Verwendung einer Resistenzkassette und der Transformation von Ashbya gossypii siehe Beispiel 6.

Das interne SalI Fragment von AgGUA2 (zwischen den Positionen 1153 und 1219) kann man durch eine wie oben skizzierte Resistenzkassette ersetzen. Das erhaltene Konstrukt erhält den Namen gua2::G418.

Das erhaltene Plasmid kann in E.coli vermehrt werden. Das XbaI / BamHI - Fragment des Konstruktes gua2::G418 kann präpariert, über Agarosegel-Elektrophorese und nachfolgende Elution der DNA aus dem Gel aufgereinigt und zur Transformation von Ashbya gossypii eingesetzt werden.

Erhaltene G418-resistente Klone können mit üblichen Methoden der PCR und Southern-Blot Analyse daraufhin untersucht werden, ob die genomische Kopie des AgGUA2 Gens tatsächlich zerstört ist. Klone, deren AgGUA2 Gen zerstört ist, sind Guanin- auxotroph.

### Beispiel 9:

### Klonierung des GAP-Promotors aus Ashbya gossypii

Das Gen für Glycerinaldehyd-3-Phosphat-Dehydrogenase aus Ashbya gossypii (AgGAP) kann man durch ein allgemein übliches Screening einer genomischen Ashbya gossypii Cosmid-Genbank (siehe Beispiel 1, mit einer Sonde, die aus Sequenzinformationen des GAP Gens aus Saccharomyces cerevisiae erstellt wurde) klonieren.

Der 5' nicht-translatierte Bereich des Gens (-373 bis -8 Region, bezogen auf den Translationsstart) wurde als Promotor angenommen. Flankierend zu dieser Sequenz wurden 2 Schnittstellen für die Restriktionsendonuklease Not1 eingeführt. In diesem Bereich findet man die bona fide TATA Box (nt 224-230), zwei Sequenzabschnitte (nt 43-51 und 77-85), die dem sogenannten GCR1 binding element und einen Sequenzabschnitt, (nt 9-20) dessen Komplement partial dem RAP1 binding element von Saccharomyces cerevisiae entspricht (siehe z.B. Johnston, M. und Carlson, M. (1992) pp.193-281 in The molecular biology and cellular biology of the yeast Saccharomyces: Gene expression, Cold Spring Harbor Laboratory Press). Die so konstruierte Promotorkassette kann als einfach portierbares Expressionsignal vor jedes beliebige Gen für die Überexpression in Ashbya gossypii gesetzt werden und führt zu deutlicher Überexpression von Genen in Ashbya gossypii, wie gezeigt in Beispiel 11.

### Beispiel 10:

### Konstruktion von Plasmiden mit Genen unter Kontrolle des GAP-Promotors aus Ashbya gossypii

Zur Einfügung der GAP-Promotorkassette 5i der kodierenden Region des AgADE4 Gens wurde nach üblichen Methode (z.B. Clover, D.M. und Hames, B.D. (1995) DNA cloning Vol.1, IRL press) 8 bp 5' des ATG Startcodons eine singuläre NotI Schnittstelle (Erkennungssequenz GCGGCCGC) eingeführt.

Die GAP-Promotorkassette kann dann über NotI in diese Position eingefügt werden. Analog kann man vorgehen bei der Klonierung der GAP-Promotorkassette 5' der kodierenden Region der Gene AgKPR1, AgGUA1, AgGUA2 sowie bei Varianten der Gene AgADE4, AgKPR1, AgGUA1 und AgGUA2.

In Ashbya gossypii wird die Expression der Gene, die die GAP-Promotorkassette 5' der kodierenden Region tragen durch den GAP-Promotor kontrolliert.

### Beispiel 11:

### Überexpression Genen in Ashbya gossypii unter Kontrolle des GAP-Promotors

Die Transformation von Ashbya gossypii mit den in Beispiel 10 beschriebenen DNA-Konstrukten kann man durchführen wie in Beispiel 6 beschrieben. Als Empfänger können bevorzugt, aber nicht ausschließlich, solche Klone dienen, die vor der hier durchzuführenden Transformation eine Disruption des zu überexprimierenden Gens tragen. So kann man z.B. die in Beispiel 6 beschriebene Mutante von Ashbya gossypii, die eine ade4::G418 Mutation trägt, mit einem in Beispiel 10 beschriebenen GAP-ADE4 Konstrukt transformieren. Man kann die Integration des Konstruktes in das Genom durch Southern-Blot-Analyse verifizieren. Die resultierenden Klone tragen kein G418 Resistenzgen mehr (sind somit G418 sensitiv) und sind Purin-prototroph. Die Überexpression kann durch Northern-Blot-Analyse oder Nachweis der enzymatischen Aktivität (wie in Beispiel 12 beschrieben) nachgewiesen werden. Bei Expression des AgADE4 Gens unter dem natürlichen Promotor kann man 0,007 U/mg Protein nachweisen. Bei Expression des AgADE4 Gens unter dem GAP Promotor kann man 0,382 U/mg Protein nachweisen

Ein Sequenzabschnitt der kodierenden Region des AgADE4 Gens kann als Sonde verwendet werden. Analog kann man mit AgKPR1, AgGUA1, AgGUA2 sowie bei Varianten all dieser Gene vorgehen. Außerdem kann man Kombinationen aus einem dieser Gene, zusammen mit anderen Genen auf diese Weise in das Genom von Ashbya gossypii einbringen.

Der Ashbya gossypii Wild-Typ hat eine spezifische PRPP Synthetase Aktivität von 22 U/mg Protein (zur Analytik der PRPP-Synthetase siehe Beispiel 13). Bei Expression des AgKPR1-Gens unter dem GAP-Promotor kann man 855 U/mg Protein nachweisen.

### Beispiel 12:

Varianten des AgADE4 Genproduktes (Glutamin-PRPP-Amidotransferase), die nicht mehr feedback durch Purine oder Zwischenprodukte der Purinsynthese gehemmt werden.

Glutamin-PRPP-Amidotransferasen werden durch Purin-Nukleotide feedback inhibiert. Diese Inhibition findet man in zahlreichen Organismen (siehe z.B. Switzer, R.L. (1989) Regulation of bacterial Glutamine Phosphoribosylpyrophosphate Amidotransferase, in: Allosteric enzymes pp. 129-151, CRC press, Boca Raton).

Die Glutamin-PRPP-Amidotransferase aus Ashbya gossypii wird ebenfalls durch AMP oder GMP gehemmt (siehe Abbildung ). Die Aktivität der Glutamin Phosphoribosylpyrophosphat Amidotransferase aus Ashbya gossypii kann man messen, wie beschrieben in Messenger und Zalkin (1979) J. Biol. Chem. 254, Seite 3382-3392.

Man kann veränderte Glutamin Phosphoribosylpyrophosphat Amidotransferasen konstruieren, die nicht mehr durch Purine gehemmt werden. Es ist offensichtlich, daß die Überexpression solch deregulierter Enzyme den Purinstoffwechsel deutlich mehr verstärken als die Überexpression der feedback inhibierten Enzyme. Veränderungen der Sequenz des AgADE4 Gens können nach üblichen Methoden (z.B. Clover, D.M. und Hames, B.D. (1995) DNA cloning Vol.1, IRL press) vorgenommen werden. Man kann z.B. folgende Aminosäuren der Glutamin Phosphoribosylpyrophosphat Amidotransferase ausgetauschen:

Das Codon, das für Aspartat an der Position 310 kodiert, kann durch ein Codon ersetzt werden, das für Valin kodiert. Das Codon, das für Lysin an der Position 333 kodiert, kann durch ein Codon ersetzt werden, das für Alanin kodiert. Das Codon, das für Alanin an der Position 417 kodiert, kann durch ein Codon ersetzt werden, das für Tryptophan kodiert. Zusätzlich können AgADE4 Gene konstruiert werden, die Kombinationen dieser Austausche tragen.

Alle Enzyme, die den D310V, den K333A, den A417W oder jede Kombination von Austauschen tragen, die D310V oder K333A enthalten, zeigen verringerte feedback Inhibition durch AMP und GMP (siehe Abbildung ). Dies kann z.B. nach Expression der Enzyme in Ashbya gossypii (siehe Beispiel 11) gezeigt werden.

### Beispiel 13:

Varianten des AgKPR1 Genproduktes (PRPP-Synthetase), die nicht mehr feedback durch Purine oder Zwischenprodukte der Purinsynthese gehemmt werden.

PRPP-Synthetasen werden feedback durch Purine, Pyrimidine und Aminosäuren inhibiert. Diese Inhibition findet man in zahlreichen Organismen (siehe z.B. Gibson, K.J. et al. (1982) J. Biol. Chem. 257, 2391-2396; Tatibana, M. et al. (1995) Adv., Enzyme Regul. 35, 229-249 und darin zitierte Arbeiten).

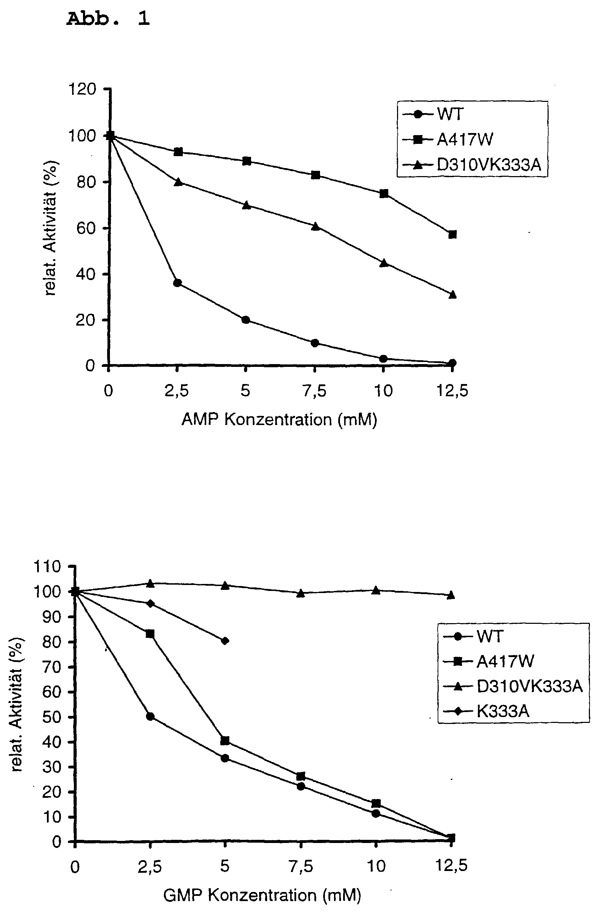

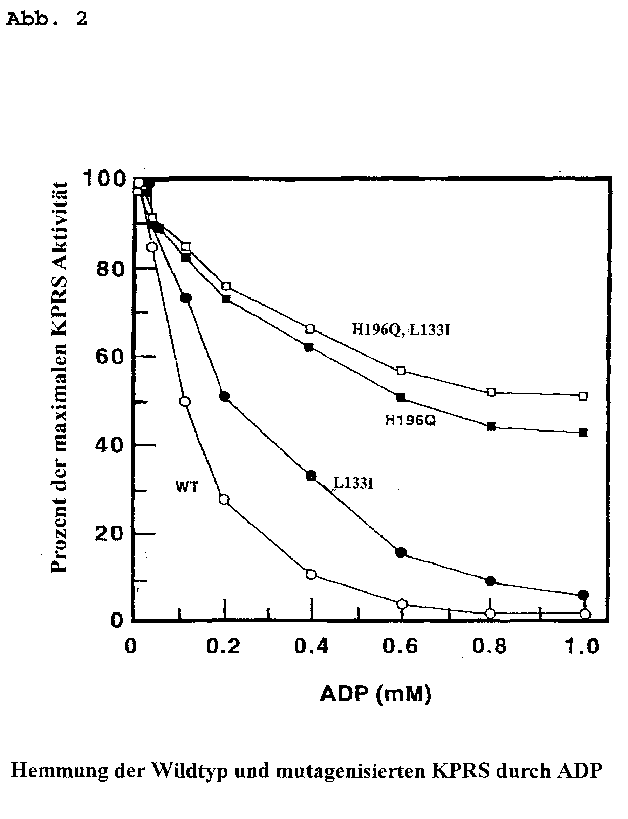

In der Forschung der klinischen Medizin sind Fälle erblicher Gicht beschrieben, deren Basis eine verstärkte Purinbiosynthese ist. Die molekulare Ursache hierfür ist eine sogenannte Superaktivität der humanen PRPP Synthetase (siehe z.B. Amer. J. Med. 55 (1973) 232-242; J. Clin. Invest. 96 (1995) 2133-2141; J. Biol. 268 (1993) 26476-26481). Die Basis hierfür kann eine Mutation sein, die dazu führt, daß das Enzym nicht mehr durch Purine feedback inhibiert wird.
Die Aktivität der PRPP Synthetase aus Ashbya gossypii kann man messen wie in Anal. Biochem. 98 (1979) 254-263 oder J. Bacteriol. 174 (1992) 6852-6856 beschrieben. Die spezifische Aktivität (U/mg) wird über die Menge an entstandenem Produkt definiert (nmol/min/mg Protein).
Man kann veränderte PRPP Synthetasen konstruieren, die nicht mehr durch Purine gehemmt werden. Es ist offensichtlich, daß die Überexpression solch deregulierter Enzyme den Purinstoffwechsel deutlich mehr verstärkt als die Überexpression der feedback inhibierten Enzyme. Veränderungen der Sequenz des AgKPR1 Gens können nach üblichen Methoden (z.B. Glover, D.M. und Hames, B.D. (1995) DNA cloning Vol. 1, IRL press) vorgenommen werden. Man kann z.B. folgende Aminosäuren der PRPP Synthetase austauschen:
Das Codon, das für Leucin an der Position 131 kodiert, kann durch ein Codon ersetzt werden, das für Isoleucin kodiert. Das Codon, das für Histidin an der Position 196 kodiert, kann durch ein Codon ersetzt werden, das für Glutamin kodiert.
Alle Enzyme, die einen dieser Aminosäureaustausche (L 131 oder H196Q) tragen, zeigen verringerte feedback Hemmung durch Purine. In Abbildung 2 ist dies gezeigt am Beispiel ADP.
Dies kann gezeigt werden, nachdem die entsprechenden Enzyme in Ashbya gossypii exprimiert wurden. Dies kann entsprechend Beispiel 11 durchgeführt werden.

### Beispiel 14:

Varianten des AgGUA1 Genproduktes (IMP-Dehydrogenase), die nicht mehr feedback durch Purine oder Zwischenprodukte der Purinsynthese gehemmt werden.

### Beispiel 15:

### Auswirkung der Verstärkung und/oder der Optimierung von Purinstoffwechsel- Enzymen und deren Gene auf die Riboflavin-Produktion in Ashbya gossypii

Man kann den Ausgangsstamm Ashbya gossypii ATCC10895, in Vergleich mit davon abgeleiteten Klonen, die chromosomale Kopien von Genen, unter Kontrolle des GAP Promotors tragen (wie in Beispiel 11 beschrieben), im Schüttelkolben auf Riboflavin- Produktivität prüfen. Man kann dazu 300 ml Schüttelkolben, mit 20 ml YPD Medium (Sambrook, J. et al. (1989) Molecular cloning: a laboratory manual, Cold Spring Harbor Laboratory Press) bei einer Inkubationstemperatur von 28°C einsetzen.

Nach 2 Tagen produziert der Kontrollstamm durchschnittlich 14,5 mg Riboflavin pro l Kulturbrühe. Stämme, die Gene für Purinstoffwechsel-Enzyme überexprimieren (wie z.B. in Beispiel 11 gezeigt) oder Gene für optimierte Purinstoffwechsel-Enzyme (z.B. wie in den Beispielen 12, 13 ,und 14 ) überexprimieren, produzieren mehr Riboflavin. So produziert der Stamm, der AgADE4D310VK333A (Beispiel 12) überexprimiert, in 2 Tagen durchschnittlich 45,4 mg Riboflavin pro l Kulturbrühe.

Der Stamm, der AgKPR1 unter dem GAP-Promotor überexprimiert, produziert statt 14 mg/l (wie der WT) 36 mg/l Riboflavin. Der Stamm, der AgKPR1H196Q unter dem GAP-Promotor überexprimiert, produziert 51 mg/l Riboflavin.

### Abbildung 1:

Messung der Aktivität der Gln-PRPP-Amidotransferase aus A. gossypii und von veränderten Formen des Enzyms in Abhängigkeit der Konzentration von Adenosin-5'-Monophosphat (AMP) und Guanosin-5'-Monophosphat (GMP).
WT: Gln-PRPP-Amidotransferase
A418W: Gln-PRPP-Amidotransferase, Alanin an Position 418 ausgetauscht gegen Tryptophan.
K333A: Gln-PRPP-Amidotransferase, Lysin an Position 333 ausgetauscht gegen Alanin.
D310VK333A: Gln-PRPP-Amidotransferase, Aspartat an Position 310 ausgetauscht gegen Valin und Lysin an Position 333 ausgetauscht gegen Alanin.

### Abbildung 2:

Messung der Aktivität der PRPP Synthetase aus A. gossypii und von veränderten Formen des Enzymes in Abhängigkeit der Konzentration von Adenosin.5'-Diphosphat (ADP)
WT: PRPP Synthetase
L131I: PRPP Synthetase, Leucin an Position 131 ausgetauscht gegen Isoleucin
H196Q: PRPP Synthetase, Histidin an Position 196 ausgetauscht gegen Glutamin
H196Q, L131I: PRPP Synthetase, Histidin an Position 196 ausgetauscht gegen Glutamin und Leucin an Position 131 ausgetauscht gegen Isoleucin

## Patentansprüche

1. Protein mit der in SEQ ID NO:2 dargestellten Polypeptidsequenz oder einer aus SEQ ID NO:2 durch Substitution, Insertion oder Deletion von bis zu 15% der Aminosäuren erhältlichen Polypeptidsequenz und der enzymatischen Aktivität einer Phosphoribosylpyrophosphat-Synthetase.

2. Protein nach Anspruch 1, das keine feed-back Hemmung durch Folgeprodukte von Stoffwechselwegen, die von Produkten des Enzyms ausgehen, mehr aufweist.

3. Protein nach Anspruch 1, das nicht mehr durch Zwischenprodukte der Purinbiosynthese, insbesondere durch Purinbasen, Purinnukleoside, Purinnukleotid-5'-Monophosphate oder Purinnukleotid-5'-Diphosphate oder Purinnukleotid-5'-Triphosphate gehemmt werden.

4. Protein nach Anspruch 1, bei dem eine oder mehrere der folgenden Aminosäureaustausche vorliegen: Lysin an Position 7 ausgetauscht gegen Valin, Aspartat an Position 52 ausgetauscht gegen Histidin, Leucin an Position 133 ausgetauscht gegen Isoleucin, Aspartat an Position 186 ausgetauscht gegen Histidin, Alanin an Position 193 ausgetauscht gegen Valin oder Histidin an Position 196 ausgetauscht gegen Glutamin.

5. Nukleinsäuresequenz codierend für ein Protein gemäß Anspruch 1.

6. Protein mit der in SEQ ID NO: 13 dargestellten Polypeptidsequenz oder einer aus SEQ ID NO: 13 durch Substitution, Insertion oder Deletion von bis zu 10% der Aminosäuren erhältlichen Polypeptidsequenz und der enzymatischen Aktivität einer Phosphoribosylpyrophosphat-Synthetase.

7. Nukleinsäuresequenz codierend für ein Protein gemäß Anspruch 6.

8. Protein mit der in SEQ ID NO:5 dargestellten Polypeptidsequenz oder einer aus SEQ ID NO:5 durch Substitution, Insertion oder Deletion von bis zu 10% der Aminosäuren erhältlichen Polypeptidsequenz und der enzymatischen Aktivität einer Glutamin-Phosphoribosylpyrophosphat-Amidotransferase.

9. Protein nach Anspruch 8, das keine feed-back Hemmung durch Folgeprodukte von Stoffwechselwegen, die von Produkten des Enzyms ausgehen, mehr aufweist.

10. Protein nach Anspruch 8, das nicht mehr durch Zwischenprodukte der Purinbiosynthese, insbesondere durch Purinbasen, Purinnukleoside, Purinnukleotid-5'-Monophosphate oder Purinnukleotid-5'-Diphosphate oder Purinnukleotid-5'-Triphosphate gehemmt werden.

11. Protein nach Anspruch 8, bei dem eine oder mehrere der folgenden Aminosäureaustausche vorliegen: Aspartat an Position 310 ausgetauscht gegen Valin, Lysin an Position 333 ausgetauscht gegen Alanin oder Alanin an Position 417 ausgetauscht gegen Tryptophan.

12. Nukleinsäuresequenz codierend für ein Protein gemäß Anspruch 8.

13. Protein mit der in SEQ ID NO:8 und 9 dargestellten Polypeptidsequenz oder einer aus SEQ ID NO:8 und 9 durch Substitution, Insertion oder Deletion von bis zu 20% der Aminosäuren erhältlichen Polypeptidsequenz und der enzymatischen Aktivität einer IMP-Dehydrogenase.

14. Protein nach Anspruch 13, das keine feed-back Hemmung durch Folgeprodukte von Stoffwechselwegen, die von Produkten des Enzyms ausgehen, mehr aufweist.

15. Protein nach Anspruch 13, das nicht mehr durch Zwischenprodukte der Purinbiosynthese, insbesondere durch Purinbasen, Purinnukleoside, Purinnukleotid-5'-Monophosphate oder Purinnukleotid-5'-Diphosphate oder Purinnukleotid-5'-Triphosphate gehemmt werden.

16. Nukleinsäuresequenz codierend für ein Protein gemäß Anspruch 13.

17. Protein mit der in SEQ ID NO:11 dargestellten Polypeptidsequenz oder einer aus SEQ ID NO:11 durch Substitution, Insertion oder Deletion von bis zu 10% der Aminosäuren erhältlichen Polypeptidsequenz und der enzymatischen Aktivität einer GMP-Synthetase.

18. Protein nach Anspruch 17, das keine feed-back Hemmung durch Folgeprodukte von Stoffwechselwegen, die von Produkten der Enzyme ausgehen, mehr aufweist.

19. Protein nach Anspruch 17, das nicht mehr durch Zwischenprodukte der Purinbiosynthese, insbesondere durch Purinbasen, Purinnukleoside, Purinnukleotid-5'-Monophosphate oder Purinnukleotid-5'-Diphosphate oder Purinnukleotid-5'-Triphosphate gehemmt werden.

20. Nukleinsäuresequenz codierend für ein Protein gemäß Anspruch 17.

21. Verwendung einer oder mehrerer der Nukleinsäuresequenzen nach den oben genannten Ansprüchen zur gentechnischen Konstruktion von Mikroorganismen, die zur Herstellung von Riboflavin in der Lage sind.

22. Verfahren zur Herstellung von Riboflavin durch Kultivierung von Mikroorganismen, die in mindestens einem Gen der Purinbiosynthese genetisch verändert worden sind.

23. Verfahren nach Anspruch 22, dadurch gekennzeichnet, daß es sich bei dem Mikroorganismus um ein Bakterium der Gattung Bacillus oder Corynebakterium handelt.

24. Verfahren nach Anspruch 22, dadurch gekennzeichnet, daß es sich bei dem Mikroorganismus um einen eukaryontischen Mikroorganismus handelt.

25. Verfahren nach Anspruch 24, dadurch gekennzeichnet, daS es sich bei dem Mikroorganismus um Ashbya gossypii handelt.

26. Verfahren nach Anspruch 24, dadurch gekennzeichnet, daß es sich bei dem Mikroorganismus um eine Hefe handelt.

27. Verfahren nach Anspruch 22, dadurch gekennzeichnet, daß es sich bei dem Mikroorganismus um eine Hefe der Gattung Candida, Saccharomyces oder Pichia handelt.

28. Verfahren nach Anspruch 22, dadurch gekennzeichnet, daß die Veränderung in mindestens einer zusätzlichen Kopie von mindestens einer der Nukleinsäuresequenzen gemäß Anspruch 5, 12, 16, 20 besteht.

29. Verfahren nach Anspruch 22, dadurch gekennzeichnet, daß durch die genetische Veränderung ein Gen codierend für ein Protein gemäß Anspruch 1, 6, 13, 17 erzeugt wird.


SEQUENZPROTOKOLL

(1) ALGEMEINE INFORMATION:

(i) ANMELDER:
(A) NAME: BASF Aktiengesellschaft
(B) STRASSE: Carl-Bosch-Strasse 38
(C) ORT: Ludwigshafen
(E) LAND: Bundesrepublik Deutschland
(F) POSTLEITZAHL: D-67056
(G) TELEPHON: 0621/6048526
(H) TELEFAX: 0621/6043123
(I) TELEX: 1762175170

(ii) ANMELDETITEL: Gene der Purinbiosynthese aus Ashbya gossypii
und deren Verwendung in der mikrobiellen
Riboflavinbiosynthese

(iii) ANZAHL DER SEQUENZEN: 13

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 1911 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: NEIN

(ix) MERKMALE:
(A) NAME/SCHLÜSSEL: 5'UTR
(B) LAGE: 1..625

(ix) MERKMALE:
(A) NAME/SCHLÜSSEL: CDS
(B) LAGE: 626..1582

(ix) MERKMALE:
(A) NAME/SCHLÜSSEL: 3'UTR
(B) LAGE: 1583..1911

(xi) SEQUENZBESCHREIBUNG: SEQ ID NO: 1:

GGTAGTCGCT CATCGACAGA CACAATCGCG TGTTCTCTCT GAATCGTCCA TTGGGTGTCA 60

GCATCCTGAT CGCGGGCGGA TGGAATGGGT AATCATTAGG AAACACCAAT GTCCCATGGT 120

ATTGTCCGTC CTCGTATGGT GTCTCAGGAG GACCCGTGAT CACGTAGTGC CACACCAGGA 180

TATTGTCTTC CTTTGGTGCT GCCACGATGT AGGGCGGGGG GTTCTCGGTC ATCATTTTGT 240

ACTCCTTTGA GAGCCGCTTG TACGCCTGTC TTGATGCCAT CTTGCCTACT ATTAGTTTCT 300

CACCACTTCC CGCCAAACAA TCTGCACTTT ACGAGCGCTA TCTATCCCTC GGGTCGCTCT 360

AGTTGATTAT TGGCGAAACT GATAGTTCAG GTACTTCCAT GATGCGGTCA TATCCACGTA 420

TGTGATCACG TGATCATCAG CCATGCTGCC AGCTCACGGG CCTGCCTACA CTATTGGAGG 480

CTCTGTGAGT CATGATTTAT TGCATATCAA GCCCAGATAG TCGTTGGGGA TACTACCGTT 540

GCCGCGATGA GCTCCGATAT TAAGTTGTAG CCAAAAATTT TAACGGATGA CTTCTTAACA 600

GTTATTGACG CCGCAATCCT ACGCC ATG TCG TCC AAT AGC ATA AAG CTG CTA 652
Met Ser Ser Asn Ser Ile Lys Leu Leu
1               5

GCA GGT AAC TCG CAC CCG GAC CTA GCT GAG AAG GTC TCC GTT CGC CTA 700
Ala Gly Asn Ser His Pro Asp Leu Ala Glu Lys Val Ser Val Arg Leu
10                  15                  20                  25

GGT GTA CCA CTT TCG AAG ATT GGA GTG TAT CAC TAC TCT AAC AAA GAG 748
Gly Val Pro Leu Ser Lys Ile Gly Val Tyr His Tyr Ser Asn Lys Glu
                30                  35                  40

ACG TCA GTT ACT ATC GGC GAA AGT ATC CGT GAT GAA GAT GTC TAC ATC 796
Thr Ser Val Thr Ile Gly Glu Ser Ile Arg Asp Glu Asp Val Tyr Ile
            45                  50                  55

ATC CAG ACA GGA ACG GGG GAG CAG GAA ATC AAC GAC TTC CTC ATG GAA 844
Ile Gln Thr Gly Thr Gly Glu Gln Glu Ile Asn Asp Phe Leu Met Glu
        60                  65                  70

CTG CTC ATC ATG ATC CAT GCC TGC CGG TCA GCC TCT GCG CGG AAG ATC 892
Leu Leu Ile Met Ile His Ala Cys Arg Ser Ala Ser Ala Arg Lys Ile
    75                  80                  85

ACA GCG GTT ATA CCA AAC TTC CCT TAC GCA AGA CAA GAC AAA AAG GAC 940
Thr Ala Val Ile Pro Asn Phe Pro Tyr Ala Arg Gln Asp Lys Lys Asp
90                  95                  100                 105

AAG TCG CGA GCA CCG ATA ACT GCC AAG CTG GTG GCC AAG ATG CTA GAG 988
Lys Ser Arg Ala Pro Ile Thr Ala Lys Leu Val Ala Lys Met Leu Glu
                110                 115                 120

ACC GCG GGG TGC AAC CAC GTT ATC ACG ATG GAT TTG CAC GCG TCT CAA 1036
Thr Ala Gly Cys Asn His Val Ile Thr Met Asp Leu His Ala Ser Gln
            125                 130                 135

ATT CAG GGT TTC TTC CAC ATT CCA GTG GAC AAC CTA TAT GCA GAG CCG 1084
Ile Gln Gly Phe Phe His Ile Pro Val Asp Asn Leu Tyr Ala Glu Pro
        140                 145                 150

AAC ATC CTG CAC TAC ATC CAA CAT AAT GTG GAC TTC CAG AAT AGT ATG 1132
Asn Ile Leu His Tyr Ile Gln His Asn Val Asp Phe Gln Asn Ser Met
    155                 160                 165

TTG GTC GCG CCA GAC GCG GGG TCG GCG AAG CGC ACG TCG ACG CTT TCG 1180
Leu Val Ala Pro Asp Ala Gly Ser Ala Lys Arg Thr Ser Thr Leu Ser
170                 175                 180                 185

GAC AAG CTG AAT CTC AAC TTC GCG TTG ATC CAC AAA GAA CGG CAG AAG 1228
Asp Lys Leu Asn Leu Asn Phe Ala Leu Ile His Lys Glu Arg Gln Lys
                190                 195                 200

GCG AAC GAG GTC TCG CGG ATG GTG TTG GTG GGT GAT GTC GCC GAC AAG 1276
Ala Asn Glu Val Ser Arg Met Val Leu Val Gly Asp Val Ala Asp Lys
            205                 210                 215

TCC TGT ATT ATT GTA GAC GAC ATG GCG GAC ACG TGC GGA ACG CTA GTG 1324
Ser Cys Ile Ile Val Asp Asp Met Ala Asp Thr Cys Gly Thr Leu Val
        220                 225                 230

AAG GCC ACT GAC ACG CTG ATC GAA AAT TGT GCG AAA GAA GTG ATT GCC 1372
Lys Ala Thr Asp Thr Leu Ile Glu Asn Cys Ala Lys Glu Val Ile Ala
    235                 240                 245

ATT GTG ACA CAC GGT ATA TTT TCT GGC GGC GCC CGC GAG AAG TTG CGC 1420
Ile Val Thr His Gly Ile Phe Ser Gly Gly Ala Arg Glu Lys Leu Arg
250                 255                 260                 265

AAC AGC AAG CTG GCA CGG ATC GTA AGC ACA AAT ACG GTG CCA GTG GAC 1468
Asn Ser Lys Leu Ala Arg Ile Val Ser Thr Asn Thr Val Pro Val Asp
                270                 275                 280

CTC AAT CTA GAT ATC TAC CAC CAA ATT GAC ATT AGT GCC ATT TTG GCC 1516
Leu Asn Leu Asp Ile Tyr His Gln Ile Asp Ile Ser Ala Ile Leu Ala
            285                 290                 295

GAG GCA ATT AGA AGG CTT CAC AAC GGG GAA AGT GTG TCG TAC CTG TTC 1564
Glu Ala Ile Arg Arg Leu His Asn Gly Glu Ser Val Ser Tyr Leu Phe
        300                 305                 310

AAT AAC GCT GTC ATG TAGTGCTGTC AGTGGCAGAT GCATGATCGC TGGCCTAATT 1619
Asn Asn Ala Val Met
    315

ATCTGTGTAA GTTGATACAA TGCAGTAAAT ACAGTACATA AAACTGAATG TTTTTCACTT 1679

AGGGGTGCTT TGTTGTTCTG ATAGCGTGTG TGCGAATTTG GAGGTGAAAG TTGAACATCA 1739

CGTAATGAAT ACAAACAAGA TTGCACATTA GGAAAAGCGA TAAATTATTT ATTATTTGCA 1799

ACTGGCCTTT GAGCGTTTAA GCCTGAACAT TTTTGCCCTT TTGTTTGACC GTACCGTTAT 1859

CACTCGTCCT TATATATGGC TATCCTTCTC TTCCGGAACT TCTTCGAGCG TA 1911

(2) INFORMATION ZU SEQ ID NO: 2:

(i) SEQUENZ CHARAKTERISTIKA:
(A) LÄNGE: 318 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 2:

Met Ser Ser Asn Ser Ile Lys Leu Leu Ala Gly Asn Ser His Pro Asp
1               5                   10                  15

Leu Ala Glu Lys Val Ser Val Arg Leu Gly Val Pro Leu Ser Lys Ile
            20                  25                  30

Gly Val Tyr His Tyr Ser Asn Lys Glu Thr Ser Val Thr Ile Gly Glu
        35                  40                  45

Ser Ile Arg Asp Glu Asp Val Tyr Ile Ile Gln Thr Gly Thr Gly Glu
    50                  55                  60

Gln Glu Ile Asn Asp Phe Leu Met Glu Leu Leu Ile Met Ile His Ala
65                  70                  75                  80

Cys Arg Ser Ala Ser Ala Arg Lys Ile Thr Ala Val Ile Pro Asn Phe
                85                  90                  95

Pro Tyr Ala Arg Gln Asp Lys Lys Asp Lys Ser Arg Ala Pro Ile Thr
            100                 105                 110

Ala Lys Leu Val Ala Lys Met Leu Glu Thr Ala Gly Cys Asn His Val
        115                 120                 125

Ile Thr Met Asp Leu His Ala Ser Gln Ile Gln Gly Phe Phe His Ile
    130                 135                 140

Pro Val Asp Asn Leu Tyr Ala Glu Pro Asn Ile Leu His Tyr Ile Gln
145                 150                 155                 160

His Asn Val Asp Phe Gln Asn Ser Met Leu Val Ala Pro Asp Ala Gly
                165                 170                 175

Ser Ala Lys Arg Thr Ser Thr Leu Ser Asp Lys Leu Asn Leu Asn Phe
            180                 185                 190

Ala Leu Ile His Lys Glu Arg Gln Lys Ala Asn Glu Val Ser Arg Met
        195                 200                 205

Val Leu Val Gly Asp Val Ala Asp Lys Ser Cys Ile Ile Val Asp Asp
    210                 215                 220

Met Ala Asp Thr Cys Gly Thr Leu Val Lys Ala Thr Asp Thr Leu Ile
225                 230                 235                 240

Glu Asn Cys Ala Lys Glu Val Ile Ala Ile Val Thr His Gly Ile Phe
                245                 250                 255

Ser Gly Gly Ala Arg Glu Lys Leu Arg Asn Ser Lys Leu Ala Arg Ile
            260                 265                 270

Val Ser Thr Asn Thr Val Pro Val Asp Leu Asn Leu Asp Ile Tyr His
        275                 280                 285

Gln Ile Asp Ile Ser Ala Ile Leu Ala Glu Ala Ile Arg Arg Leu His
    290                 295                 300

Asn Gly Glu Ser Val Ser Tyr Leu Phe Asn Asn Ala Val Met
305                 310                 315

(2) INFORMATION ZU SEQ ID NO: 3:

     (i) SEQUENZ CHARAKTERISTIKA:
          (A) LÄNGE: 5369 Basenpaare
          (B) ART: Nukleinsäure
          (C) STRANGFORM: Einzel
          (D) TOPOLOGIE: linear

    (ii) ART DES MOLEKÜLS: DNS (genomisch)

   (iii) HYPOTHETISCH: NEIN

   (iii) ANTISENSE: NEIN

    (ix) MERKMALE:
          (A) NAME/SCHLÜSSEL: 5'UTR
          (B) LAGE: 1..54

    (ix) MERKMALE:
          (A) NAME/SCHLÜSSEL: CDS
          (B) LAGE: 55..1482

    (ix) MERKMALE:
          (A) NAME/SCHLÜSSEL: CDS
          (B) LAGE: 1767..3299

    (ix) MERKMALE:
          (A) NAME/SCHLÜSSEL: CDS
          (B) LAGE: 3588..4703

(ix) MERKMALE:
(A) NAME/SCHLÜSSEL: 3'UTR
(B) LAGE: 4704..5369

(xi) SEQUENZBESCHREIBUNG: SEQ ID NO: 3:

AAGCTTGACC TTGGCTGGCA CTTGAGTCGG CAGACAGGTG GACTAACCCG AGCA ATG 57
Met
1

GAT CGT GGT TGT AAA GGT ATC TCT TAT GTG CTC AGT GCA ATG GTT TTT 105
Asp Arg Gly Cys Lys Gly Ile Ser Tyr Val Leu Ser Ala Met Val Phe
            5                   10                  15

CAC ATA ATA CCG ATT ACA TTT GAA ATA TCG ATG GTA TGT GGC ATA TTG 153
His Ile Ile Pro Ile Thr Phe Glu Ile Ser Met Val Cys Gly Ile Leu
        20                  25                  30

ACA TAC CAG TTT GGT GCT TCC TTC GCT GCT ATA ACA TTC TCG ACT ATG 201
Thr Tyr Gln Phe Gly Ala Ser Phe Ala Ala Ile Thr Phe Ser Thr Met
    35                  40                  45

CTT CTT TAC TCC ATC TTT ACT TTC AGA ACG ACG GCG TGG CGC ACA CGG 249
Leu Leu Tyr Ser Ile Phe Thr Phe Arg Thr Thr Ala Trp Arg Thr Arg
50                  55                  60                  65

TTT AGG CGT GAT GCG AAC AAG GCT GAC AAT AAG GCC GCT AGT GTG GCA 297
Phe Arg Arg Asp Ala Asn Lys Ala Asp Asn Lys Ala Ala Ser Val Ala
                70                  75                  80

TTG GAT TCC CTA ATA AAT TTT GAA GCT GTA AAG TAT TTC AAT AAC GAG 345
Leu Asp Ser Leu Ile Asn Phe Glu Ala Val Lys Tyr Phe Asn Asn Glu
            85                  90                  95

AAG TAC CTT GCG GAC AAG TAT CAC ACA TCC TTG ATG AAG TAC CGG GAT 393
Lys Tyr Leu Ala Asp Lys Tyr His Thr Ser Leu Met Lys Tyr Arg Asp
        100                 105                 110

TCC CAG ATA AAG GTC TCG CAA TCG CTG GCG TTT TTG AAC ACC GGC CAG 441
Ser Gln Ile Lys Val Ser Gln Ser Leu Ala Phe Leu Asn Thr Gly Gln
    115                 120                 125

AAC CTA ATT TTT ACC ACT GCA CTG ACT GCA ATG ATG TAT ATG GCC TGT 489
Asn Leu Ile Phe Thr Thr Ala Leu Thr Ala Met Met Tyr Met Ala Cys
130                 135                 140                 145

AAT GGT GTT ATG CAG GGC TCT CTT ACA GTG GGG GAT CTT GTG TTA ATT 537
Asn Gly Val Met Gln Gly Ser Leu Thr Val Gly Asp Leu Val Leu Ile
                150                 155                 160

AAT CAA CTG GTA TTC CAG CTC TCC GTG CCA CTA AAC TTC CTT GGT AGC 585
Asn Gln Leu Val Phe Gln Leu Ser Val Pro Leu Asn Phe Leu Gly Ser
            165                 170                 175

GTC TAC CGT GAT CTC AAG CAG TCT CTG ATA GAT ATG GAA TCT TTA TTT 633
Val Tyr Arg Asp Leu Lys Gln Ser Leu Ile Asp Met Glu Ser Leu Phe
        180                 185                 190

AAA CTG CAA AAA AAT CAG GTC ACA ATT AAG AAC TCC CCA AAT GCC CAG 681
Lys Leu Gln Lys Asn Gln Val Thr Ile Lys Asn Ser Pro Asn Ala Gln
    195                 200                 205

AAC CTA CCA ATA CAC AAA CCG TTG GAT ATT CGC TTT GAA AAT GTT ACG 729
Asn Leu Pro Ile His Lys Pro Leu Asp Ile Arg Phe Glu Asn Val Thr
210                 215                 220                 225

TTT GGC TAT GAC CCG GAG CGG CGT ATA TTG AAC AAT GTT TCG TTT ACC 777
Phe Gly Tyr Asp Pro Glu Arg Arg Ile Leu Asn Asn Val Ser Phe Thr
                230                 235                 240

ATC CCA GCT GGA ATG AAG ACT GCC ATA GTA GGC CCA TCG GGC TCG GGG 825
Ile Pro Ala Gly Met Lys Thr Ala Ile Val Gly Pro Ser Gly Ser Gly
            245                 250                 255

AAG TCC ACC ATT TTG AAG CTC GTA TTT AGA TTC TAT GAG CCC GAG CAA 873
Lys Ser Thr Ile Leu Lys Leu Val Phe Arg Phe Tyr Glu Pro Glu Gln
        260                 265                 270

GGT CGT ATC CTA GTT GGC GGC ACA GAT ATC CGC GAT TTA GAC TTG CTT 921
Gly Arg Ile Leu Val Gly Gly Thr Asp Ile Arg Asp Leu Asp Leu Leu
    275                 280                 285

TCT TTA CGG AAG GCT ATC GGT GTC GTG CCC CAA GAT ACT CCT CTC TTC 969
Ser Leu Arg Lys Ala Ile Gly Val Val Pro Gln Asp Thr Pro Leu Phe
290                 295                 300                 305

AAT GAC ACA ATC TGG GAG AAT GTT AAA TTC GGC AAT ATC AGT TCC TCT 1017
Asn Asp Thr Ile Trp Glu Asn Val Lys Phe Gly Asn Ile Ser Ser Ser
                310                 315                 320

GAC GAT GAG ATT CTC AGG GCC ATA GAA AAA GCT CAA CTC ACG AAG CTA 1065
Asp Asp Glu Ile Leu Arg Ala Ile Glu Lys Ala Gln Leu Thr Lys Leu
            325                 330                 335

CTC CAG AAC CTA CCA AAG GGC GCT TCC ACC GTT GTA GGG GAG CGC GGT 1113
Leu Gln Asn Leu Pro Lys Gly Ala Ser Thr Val Val Gly Glu Arg Gly
        340                 345                 350

TTG ATG ATC AGC GGA GGT GAG AAA CAA AGG CTT GCT ATT GCT CGT GTG 1161
Leu Met Ile Ser Gly Gly Glu Lys Gln Arg Leu Ala Ile Ala Arg Val
    355                 360                 365

CTT TTG AAG GAC GCT CCG CTG ATG TTT TTC GAC GAG GCT ACA AGT GCT 1209
Leu Leu Lys Asp Ala Pro Leu Met Phe Phe Asp Glu Ala Thr Ser Ala
370                 375                 380                 385

CTG GAT ACA CAC ACA GAG CAG GCA CTC TTG CAC ACC ATT CAG CAG AAC 1257
Leu Asp Thr His Thr Glu Gln Ala Leu Leu His Thr Ile Gln Gln Asn
                390                 395                 400

TTT TCT TCC AAT TCA AAG ACG AGC GTT TAC GTT GCC CAT AGA CTG CGC 1305
Phe Ser Ser Asn Ser Lys Thr Ser Val Tyr Val Ala His Arg Leu Arg
            405                 410                 415

ACA ATC GCT GAT GCA GAT AAG ATC ATT GTT CTT GAA CAA GGT TCT GTC 1353
Thr Ile Ala Asp Ala Asp Lys Ile Ile Val Leu Glu Gln Gly Ser Val
        420                 425                 430

CGC GAA GAG GGC ACA CAC AGC TCG CTG TTA GCG TCA CAA GGA TCC CTA 1401
Arg Glu Glu Gly Thr His Ser Ser Leu Leu Ala Ser Gln Gly Ser Leu
    435                 440                 445

TAC CGG GGT CTG TGG GAT ATT CAG GAA AAC CTA ACG CTT CCG GAA CGG 1449
Tyr Arg Gly Leu Trp Asp Ile Gln Glu Asn Leu Thr Leu Pro Glu Arg
450                 455                 460                 465

CCT GAG CAG TCA ACC GGA TCT CAG CAT GCA TAGACGTCTG ACTAGAGATT 1499
Pro Glu Gln Ser Thr Gly Ser Gln His Ala
                470                 475

ATATAATAAC CCTCGAGCCA AAATTATACG GCGCTAACAA GTAAAAATTT TAGTTACTTT 1559

TCTGACTTCT CTACGCTGAC TTCTCTACCC TTCTAACATA GTTAATTGAA GTAGTGGTTA 1619

ATGACGACTG CATTTTATTA TTGTCCACTT TGCATTAGAA GTACTAGTGC TTAAGCGCTC 1679

TTTAGGCCGC TTTCTTCTTC TTTGTCAGGC CGCAAGGTAA AGGAAGCACC AACGGATTGC 1739

TACCGCTGCT ATTCCTGCTC TCTCAAG ATG TGT GGC ATA TTA GGC GTT GTG 1790
Met Cys Gly Ile Leu Gly Val Val
1 5

CTA GCC GAT CAG TCG AAG GTG GTC GCC CCT GAG TTG TTT GAT GGC TCA 1838
Leu Ala Asp Gln Ser Lys Val Val Ala Pro Glu Leu Phe Asp Gly Ser
10 15 20

CTG TTC TTA CAG CAT CGC GGT CAA GAT GCT GCC GGG ATT GCT ACG TGC 1886
Leu Phe Leu Gln His Arg Gly Gln Asp Ala Ala Gly Ile Ala Thr Cys
25 30 35 40

GGC CCC GGT GGG CGC TTG TAC CAA TGT AAG GGC AAT GGT ATG GCA CGG 1934
Gly Pro Gly Gly Arg Leu Tyr Gln Cys Lys Gly Asn Gly Met Ala Arg
45 50 55

GAC GTG TTC ACG CAA GCT CGG ATG TCA GGG TTG GTT GGC TCT ATG GGG 1982
Asp Val Phe Thr Gln Ala Arg Met Ser Gly Leu Val Gly Ser Met Gly
60 65 70

ATT GCA CAC CTG AGA TAT CCC ACT GCA GGC TCC AGT GCG AAC TCA GAA 2030
Ile Ala His Leu Arg Tyr Pro Thr Ala Gly Ser Ser Ala Asn Ser Glu
75 80 85

GCG CAG CCA TTC TAT GTG AAT AGT CCC TAC GGA ATT TGC ATG AGT CAT 2078
Ala Gln Pro Phe Tyr Val Asn Ser Pro Tyr Gly Ile Cys Met Ser His
90 95 100

AAT GGT AAT CTG GTG AAC ACG ATG TCT CTA CGT AGA TAT CTT GAT GAA 2126
Asn Gly Asn Leu Val Asn Thr Met Ser Leu Arg Arg Tyr Leu Asp Glu
105 110 115 120

GAC GTT CAC CGT CAT ATT AAC ACG GAC AGC GAT TCT GAG CTA CTG CTT 2174
Asp Val His Arg His Ile Asn Thr Asp Ser Asp Ser Glu Leu Leu Leu
125 130 135

AAT ATA TTT GCC GCG GAG CTG GAA AAG TAC AAC AAA TAT CGT GTG AAC 2222
Asn Ile Phe Ala Ala Glu Leu Glu Lys Tyr Asn Lys Tyr Arg Val Asn
140 145 150

AAC GAT GAT ATA TTT TGT GCT CTA GAG GGT GTT TAC AAA CGT TGT CGC 2270
Asn Asp Asp Ile Phe Cys Ala Leu Glu Gly Val Tyr Lys Arg Cys Arg
155 160 165

GGT GGC TAT GCT TGT GTT GGC ATG TTG GCG GGA TAT GGA TTG TTT GGT 2318
Gly Gly Tyr Ala Cys Val Gly Met Leu Ala Gly Tyr Gly Leu Phe Gly
170 175 180

TTC CGG GAC CCC AAT GGG ATC AGG CCG CTA TTG TTT GGT GAG CGC GTC 2366
Phe Arg Asp Pro Asn Gly Ile Arg Pro Leu Leu Phe Gly Glu Arg Val
185 190 195 200

AAC GAT GAC GGC ACC ATG GAC TAC ATG CTA GCG TCC GAA AGT GTC GTT 2414
Asn Asp Asp Gly Thr Met Asp Tyr Met Leu Ala Ser Glu Ser Val Val
205 210 215

CTT AAG GCC CAC CGC TTC CAA AAC ATA CGT GAT ATT CTT CCC GGC CAA 2462
Leu Lys Ala His Arg Phe Gln Asn Ile Arg Asp Ile Leu Pro Gly Gln
220 225 230

GCC GTC ATT ATC CCT AAA ACG TGC GGC TCC AGT CCA CCA GAG TTC CGG 2510
Ala Val Ile Ile Pro Lys Thr Cys Gly Ser Ser Pro Pro Glu Phe Arg
235 240 245

CAG GTA GTG CCA ATT GAG GCC TAC AAA CCG GAC TTG TTT GAG TAC GTG 2558
Gln Val Val Pro Ile Glu Ala Tyr Lys Pro Asp Leu Phe Glu Tyr Val
250 255 260

TAT TTC GCT CGT GCT GAC AGC GTT CTG GAC GGT ATT TCC GTT TAC CAT 2606
Tyr Phe Ala Arg Ala Asp Ser Val Leu Asp Gly Ile Ser Val Tyr His
265 270 275 280

ACA CGC CTG TTG ATG GGT ATC AAA CTT GCC GAG AAC ATC AAA AAA CAG 2654
Thr Arg Leu Leu Met Gly Ile Lys Leu Ala Glu Asn Ile Lys Lys Gln
285 290 295

ATC GAT CTG GAC GAA ATT GAC GTT GTT GTA TCT GTT CCT GAC ACT GCA 2702
Ile Asp Leu Asp Glu Ile Asp Val Val Val Ser Val Pro Asp Thr Ala
300 305 310

CGT ACC TGT GCA TTG GAG TGT GCC AAC CAT TTA AAC AAA CCT TAT CGC 2750
Arg Thr Cys Ala Leu Glu Cys Ala Asn His Leu Asn Lys Pro Tyr Arg
315 320 325

GAA GGA TTT GTC AAG AAC AGA TAT GTT GGA AGA ACA TTT ATC ATG CCA 2798
Glu Gly Phe Val Lys Asn Arg Tyr Val Gly Arg Thr Phe Ile Met Pro
330 335 340

AAC CAA AAA GAG CGA GTA TCT TCT GTG CGC CGC AAG TTG AAC CCA ATG 2846
Asn Gln Lys Glu Arg Val Ser Ser Val Arg Arg Lys Leu Asn Pro Met
345 350 355 360

AAC TCA GAA TTT AAA GAC AAG CGC GTG CTG ATT GTC GAT GAT TCC ATT 2894
Asn Ser Glu Phe Lys Asp Lys Arg Val Leu Ile Val Asp Asp Ser Ile
365 370 375

GTG CGA GGT ACC ACT TCC AAA GAG ATT GTT AAC ATG GCG AAG GAA TCC 2942
Val Arg Gly Thr Thr Ser Lys Glu Ile Val Asn Met Ala Lys Glu Ser
380 385 390

GGT GCT GCC AAG GTC TAC TTT GCC TCT GCA GCG CCA GCA ATT CGT TTC 2990
Gly Ala Ala Lys Val Tyr Phe Ala Ser Ala Ala Pro Ala Ile Arg Phe
395 400 405

AAT CAC ATC TAC GGG ATT GAC CTA GCA GAT ACT AAG CAG CTT GTC GCC 3038
Asn His Ile Tyr Gly Ile Asp Leu Ala Asp Thr Lys Gln Leu Val Ala
410 415 420

TAC AAC AGA ACT GTT GAA GAA ATC ACT GCG GAG CTG GGC TGT GAC CGC 3086
Tyr Asn Arg Thr Val Glu Glu Ile Thr Ala Glu Leu Gly Cys Asp Arg
425 430 435 440

GTC ATC TAT CAA TCT TTG GAT GAC CTC ATC GAC TGT TGC AAG ACA GAC 3134
Val Ile Tyr Gln Ser Leu Asp Asp Leu Ile Asp Cys Cys Lys Thr Asp
445 450 455

ATC ATC TCA GAA TTT GAA GTT GGA GTT TTC ACT GGT AAC TAC GTT ACA 3182
Ile Ile Ser Glu Phe Glu Val Gly Val Phe Thr Gly Asn Tyr Val Thr
460 465 470

GGT GTT GAG GAT GTG TAC TTG CAG GAA TTA GAA CGT TGC CGC GCT CTT 3230
Gly Val Glu Asp Val Tyr Leu Gln Glu Leu Glu Arg Cys Arg Ala Leu
475 480 485

AAT AAC TCG AAT AAG GGT GAA GCG AAG GCC GAG GTT GAT ATT GGT CTC     3278
Asn Asn Ser Asn Lys Gly Glu Ala Lys Ala Glu Val Asp Ile Gly Leu
    490                 495                 500

TAC AAT TCT GCC GAC TAT TAGCGGCGCC GTTGCCGGCA TCCGGCCCCA     3326
Tyr Asn Ser Ala Asp Tyr
505                 510

TATATAGACT CATCGGGACC TAAAATAAGC CTTTACAGAT CATTATCTAC AAATATAGAT     3386

ACCATTAAAA GCCTGACTTT CGACTTACTC CTAGCACACC CCGTTGTATC CCTGTGCTTG     3446

CTTTCTTAAA TGCCGTTGGT TAGGCTTTGG ACTTAGCGTC CCGCCCATTT TCTAGCATGT     3506

GCAGATCTAG CAAATTTGGC CTAAGACAAG AAGATCCATT CGGCACCCAC ATCCTGGAGC     3566

CAGCACACAG TGGACCCAGA C ATG AGC AGC GGC AAT ATA TGG AAG CAA TTG     3617
                        Met Ser Ser Gly Asn Ile Trp Lys Gln Leu
                          1               5                  10

CTA GAG GAG AAT AGC GAA CAG CTG GAC CAG TCC ACT ACG GAG ACT TAC     3665
Leu Glu Glu Asn Ser Glu Gln Leu Asp Gln Ser Thr Thr Glu Thr Tyr
                 15                  20                  25

GTG GTA TGC TGC GAG AAC GAA GAT TCC CTT AAC CAG TTT TTG CAA CAA     3713
Val Val Cys Cys Glu Asn Glu Asp Ser Leu Asn Gln Phe Leu Gln Gln
             30                  35                  40

TGT TGG CAG ATT GAC GAG GGC GAG AAG GTG ACC AAC CTG GAG CCG TTG     3761
Cys Trp Gln Ile Asp Glu Gly Glu Lys Val Thr Asn Leu Glu Pro Leu
         45                  50                  55

GGA TTC TTT ACA AAG GTG GTT TCG CGC GAC GAA GAG AAC CTC CGG CTC     3809
Gly Phe Phe Thr Lys Val Val Ser Arg Asp Glu Glu Asn Leu Arg Leu
     60                  65                  70

AAC GTA TAC TAT GCC AAG AGC CCA CTG GAT GCA CAG ACG CTG CAG TTT     3857
Asn Val Tyr Tyr Ala Lys Ser Pro Leu Asp Ala Gln Thr Leu Gln Phe
 75                  80                  85                  90

CTG GGC GTG TTC CTG CGC CAA ATG GAA ACC TCA CAA ATA CGT TGG ATC     3905
Leu Gly Val Phe Leu Arg Gln Met Glu Thr Ser Gln Ile Arg Trp Ile
                 95                 100                 105

TTC CTA CTG GAC TGG CTG CTA GAC GAT AAA CGA TTA TGG CTA CGT CAA     3953
Phe Leu Leu Asp Trp Leu Leu Asp Asp Lys Arg Leu Trp Leu Arg Gln
            110                 115                 120

CTG CGG AAC TCG TGG GCC GCC TTG GAG GAA GCG CAG GTG GCA CCC TTT     4001
Leu Arg Asn Ser Trp Ala Ala Leu Glu Glu Ala Gln Val Ala Pro Phe
        125                 130                 135

CCA GGT GGC GCT GTG GTG GTG GTC CTC AAC CCG AGT CAC GTG ACA CAA 4049
Pro Gly Gly Ala Val Val Val Val Leu Asn Pro Ser His Val Thr Gln
140 145 150

CTG GAG CGA AAC ACG ATG GTT TGG AAC TCC CGC CGT CTG GAC CTG GTA 4097
Leu Glu Arg Asn Thr Met Val Trp Asn Ser Arg Arg Leu Asp Leu Val
155 160 165 170

CAC CAG ACA CTG CGA GCT GCA TGC CTC AAC ACC GGC TCG GCG CTA GTT 4145
His Gln Thr Leu Arg Ala Ala Cys Leu Asn Thr Gly Ser Ala Leu Val
175 180 185

ACA CTT GAT CCT AAT ACT GCG CGC GAA GAC GTC ATG CAC ATA TGT GCG 4193
Thr Leu Asp Pro Asn Thr Ala Arg Glu Asp Val Met His Ile Cys Ala
190 195 200

CTG CTT GCG GGG CTG CCT ACA TCC CGT CCC GTC GCG ATG CTA AGC CTG 4241
Leu Leu Ala Gly Leu Pro Thr Ser Arg Pro Val Ala Met Leu Ser Leu
205 210 215

CAA AGT CTA TTC ATC CCC CAC GGT GCA GAT TCC ATC GGC AAG ATC TGC 4289
Gln Ser Leu Phe Ile Pro His Gly Ala Asp Ser Ile Gly Lys Ile Cys
220 225 230

ACC ATC GCG CCC GAG TTC CCT GTT GCT ACG GTG TTC GAC AAC GAT TTT 4337
Thr Ile Ala Pro Glu Phe Pro Val Ala Thr Val Phe Asp Asn Asp Phe
235 240 245 250

GTG AGC TCG ACA TTC GAG GCC GCA ATT GCT CCA GAA CTT ACT CCA GGA 4385
Val Ser Ser Thr Phe Glu Ala Ala Ile Ala Pro Glu Leu Thr Pro Gly
255 260 265

CCA CGT GTG CCA TCT GAC CAC CCA TGG CTA ACA GAG CCT ACC AAC CCC 4433
Pro Arg Val Pro Ser Asp His Pro Trp Leu Thr Glu Pro Thr Asn Pro
270 275 280

CCT TCG GAG GCA ACC GCT TGG CAT TTC GAT CTC CAA GGT CGC CTC GCT 4481
Pro Ser Glu Ala Thr Ala Trp His Phe Asp Leu Gln Gly Arg Leu Ala
285 290 295

ACC CTA TAC CGG CAT CTT GGT GAC TCT AAC AAG GCC ATA TCT GTT ACT 4529
Thr Leu Tyr Arg His Leu Gly Asp Ser Asn Lys Ala Ile Ser Val Thr
300 305 310

CAG CAC CGC TTC CAC AAG CCC CGC TCG GAA GAT TAT GCA TAC GAA TTC 4577
Gln His Arg Phe His Lys Pro Arg Ser Glu Asp Tyr Ala Tyr Glu Phe
315 320 325 330

GAG CTG CCG TCT AAG CAC CCT ACA ATA CGT GAC CTC ATA CGC TCT GCC 4625
Glu Leu Pro Ser Lys His Pro Thr Ile Arg Asp Leu Ile Arg Ser Ala
335 340 345

GCA GCC GAC TCA CCG AAC GAC GTC GCT GAC TCC ATC GAT GGG CTT ATG 4673
Ala Ala Asp Ser Pro Asn Asp Val Ala Asp Ser Ile Asp Gly Leu Met
350 355 360

GAT GGT ATC GTA CAA AGG AAT GTT CAT TGACGTCGAC ACAAAAATTT 4720
Asp Gly Ile Val Gln Arg Asn Val His
365 370

TGTTACTGTT CTCTCGAGAA CTATTCTCAT CCAGTACTGA CATATTAGAA GGCGAAGTGA 4780

ACTAGGATTT ATATAAAGTA GCCTTCAGGC AATTGCACAG GGTCTATTGA GTCGCTGCCG 4840

TTCACGAGAG AGCCCAATAT ATCGAGGACT AATTGGTCAC TTTTGTTTTG CTATACTCAC 4900

CCTGTATTTG CTAATCATTT ATCCGCTTTG TCCAAGTGGT TGCGAAGATA TCGAGCCAGA 4960

ACATTAGAAT CTGGTTTGCC GCATCCTAGA GCTGTCTCCA AGCCAGTTGA ACCGTTGCGG 5020

GAGATTACCG CAGCCGGTTT GATCAGAGTA CTGGTGACTG CCAGCACCCA CGTTTGTGAC 5080

TTATAAATAT ACGCCCTGTG GAGCCATAGC CATTGGCATA AAGAGAAGAG CACCCCGTGC 5140

CACGATGCAG ACACTTCCGG TGTACCCAGC GTCACAGACT GCGTCGCCTA CGAAGCGTGA 5200

ACTTGCAGCG GCGCCCTCGG TGCCGCAGGA CGGCGCCCGG CTGCCTGCGC AGCTCACTTT 5260

AGTGACGCCC CCAGAACCTG ATATCCAGAA GAAGTCAGTG CGATCTCAGG TCGCGCGTTT 5320

AAGCATCTCG GAGACAGATG TAGTGAAGAG TGATATCGTG GCTAAGCTT 5369

(2) INFORMATION ZU SEQ ID NO: 4:

(i) SEQUENZ CHARAKTERISTIKA:
(A) LÄNGE: 475 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 4:

Met Asp Arg Gly Cys Lys Gly Ile Ser Tyr Val Leu Ser Ala Met Val
1               5                   10                  15

Phe His Ile Ile Pro Ile Thr Phe Glu Ile Ser Met Val Cys Gly Ile
            20                  25                  30

Leu Thr Tyr Gln Phe Gly Ala Ser Phe Ala Ala Ile Thr Phe Ser Thr
        35                  40                  45

Met Leu Leu Tyr Ser Ile Phe Thr Phe Arg Thr Thr Ala Trp Arg Thr
    50                  55                  60

Arg Phe Arg Arg Asp Ala Asn Lys Ala Asp Asn Lys Ala Ala Ser Val
65                  70                  75                  80

Ala Leu Asp Ser Leu Ile Asn Phe Glu Ala Val Lys Tyr Phe Asn Asn
                85                  90                  95

Glu Lys Tyr Leu Ala Asp Lys Tyr His Thr Ser Leu Met Lys Tyr Arg
            100                 105                 110

Asp Ser Gln Ile Lys Val Ser Gln Ser Leu Ala Phe Leu Asn Thr Gly
        115                 120                 125

Gln Asn Leu Ile Phe Thr Thr Ala Leu Thr Ala Met Met Tyr Met Ala
    130                 135                 140

Cys Asn Gly Val Met Gln Gly Ser Leu Thr Val Gly Asp Leu Val Leu
145                 150                 155                 160

Ile Asn Gln Leu Val Phe Gln Leu Ser Val Pro Leu Asn Phe Leu Gly
                165                 170                 175

Ser Val Tyr Arg Asp Leu Lys Gln Ser Leu Ile Asp Met Glu Ser Leu
            180                 185                 190

Phe Lys Leu Gln Lys Asn Gln Val Thr Ile Lys Asn Ser Pro Asn Ala
        195                 200                 205

Gln Asn Leu Pro Ile His Lys Pro Leu Asp Ile Arg Phe Glu Asn Val
    210                 215                 220

Thr Phe Gly Tyr Asp Pro Glu Arg Arg Ile Leu Asn Asn Val Ser Phe
225                 230                 235                 240

Thr Ile Pro Ala Gly Met Lys Thr Ala Ile Val Gly Pro Ser Gly Ser
                245                 250                 255

Gly Lys Ser Thr Ile Leu Lys Leu Val Phe Arg Phe Tyr Glu Pro Glu
            260                 265                 270

Gln Gly Arg Ile Leu Val Gly Gly Thr Asp Ile Arg Asp Leu Asp Leu
        275                 280                 285

Leu Ser Leu Arg Lys Ala Ile Gly Val Val Pro Gln Asp Thr Pro Leu
    290                 295                 300

Phe Asn Asp Thr Ile Trp Glu Asn Val Lys Phe Gly Asn Ile Ser Ser
305                 310                 315                 320

Ser Asp Asp Glu Ile Leu Arg Ala Ile Glu Lys Ala Gln Leu Thr Lys
                325                 330                 335

Leu Leu Gln Asn Leu Pro Lys Gly Ala Ser Thr Val Val Gly Glu Arg
            340                 345                 350

Gly Leu Met Ile Ser Gly Gly Glu Lys Gln Arg Leu Ala Ile Ala Arg
        355                 360                 365

Val Leu Leu Lys Asp Ala Pro Leu Met Phe Phe Asp Glu Ala Thr Ser
    370                 375                 380

Ala Leu Asp Thr His Thr Glu Gln Ala Leu Leu His Thr Ile Gln Gln
385                 390                 395                 400

Asn Phe Ser Ser Asn Ser Lys Thr Ser Val Tyr Val Ala His Arg Leu
                405                 410                 415

Arg Thr Ile Ala Asp Ala Asp Lys Ile Ile Val Leu Glu Gln Gly Ser
            420                 425                 430

Val Arg Glu Glu Gly Thr His Ser Ser Leu Leu Ala Ser Gln Gly Ser
        435                 440                 445

Leu Tyr Arg Gly Leu Trp Asp Ile Gln Glu Asn Leu Thr Leu Pro Glu
    450                 455                 460

Arg Pro Glu Gln Ser Thr Gly Ser Gln His Ala
465                 470                 475

(2) INFORMATION ZU SEQ ID NO: 5:

(i) SEQUENZ CHARAKTERISTIKA:
(A) LÄNGE: 510 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 5:

Met Cys Gly Ile Leu Gly Val Val Leu Ala Asp Gln Ser Lys Val Val
1               5                   10                  15

Ala Pro Glu Leu Phe Asp Gly Ser Leu Phe Leu Gln His Arg Gly Gln
            20                  25                  30

Asp Ala Ala Gly Ile Ala Thr Cys Gly Pro Gly Gly Arg Leu Tyr Gln
        35                  40                  45

Cys Lys Gly Asn Gly Met Ala Arg Asp Val Phe Thr Gln Ala Arg Met
    50                  55                  60

Ser Gly Leu Val Gly Ser Met Gly Ile Ala His Leu Arg Tyr Pro Thr
65                  70                  75                  80

Ala Gly Ser Ser Ala Asn Ser Glu Ala Gln Pro Phe Tyr Val Asn Ser
                85                  90                  95

Pro Tyr Gly Ile Cys Met Ser His Asn Gly Asn Leu Val Asn Thr Met
            100                 105                 110

Ser Leu Arg Arg Tyr Leu Asp Glu Asp Val His Arg His Ile Asn Thr
        115                 120                 125

Asp Ser Asp Ser Glu Leu Leu Leu Asn Ile Phe Ala Ala Glu Leu Glu
    130                 135                 140

Lys Tyr Asn Lys Tyr Arg Val Asn Asn Asp Asp Ile Phe Cys Ala Leu
145                 150                 155                 160

Glu Gly Val Tyr Lys Arg Cys Arg Gly Gly Tyr Ala Cys Val Gly Met
                165                 170                 175

Leu Ala Gly Tyr Gly Leu Phe Gly Phe Arg Asp Pro Asn Gly Ile Arg
            180                 185                 190

Pro Leu Leu Phe Gly Glu Arg Val Asn Asp Asp Gly Thr Met Asp Tyr
        195                 200                 205

Met Leu Ala Ser Glu Ser Val Val Leu Lys Ala His Arg Phe Gln Asn
    210                 215                 220

Ile Arg Asp Ile Leu Pro Gly Gln Ala Val Ile Ile Pro Lys Thr Cys
225                 230                 235                 240

Gly Ser Ser Pro Pro Glu Phe Arg Gln Val Val Pro Ile Glu Ala Tyr
                245                 250                 255

Lys Pro Asp Leu Phe Glu Tyr Val Tyr Phe Ala Arg Ala Asp Ser Val
            260                 265                 270

Leu Asp Gly Ile Ser Val Tyr His Thr Arg Leu Leu Met Gly Ile Lys
        275                 280                 285

Leu Ala Glu Asn Ile Lys Lys Gln Ile Asp Leu Asp Glu Ile Asp Val
    290                 295                 300

Val Val Ser Val Pro Asp Thr Ala Arg Thr Cys Ala Leu Glu Cys Ala
305                 310                 315                 320

Asn His Leu Asn Lys Pro Tyr Arg Glu Gly Phe Val Lys Asn Arg Tyr
                325                 330                 335

Val Gly Arg Thr Phe Ile Met Pro Asn Gln Lys Glu Arg Val Ser Ser
            340                 345                 350

Val Arg Arg Lys Leu Asn Pro Met Asn Ser Glu Phe Lys Asp Lys Arg
        355                 360                 365

Val Leu Ile Val Asp Asp Ser Ile Val Arg Gly Thr Thr Ser Lys Glu
    370                 375                 380

Ile Val Asn Met Ala Lys Glu Ser Gly Ala Ala Lys Val Tyr Phe Ala
385                 390                 395                 400

Ser Ala Ala Pro Ala Ile Arg Phe Asn His Ile Tyr Gly Ile Asp Leu
                405                 410                 415

Ala Asp Thr Lys Gln Leu Val Ala Tyr Asn Arg Thr Val Glu Glu Ile
            420                 425                 430

Thr Ala Glu Leu Gly Cys Asp Arg Val Ile Tyr Gln Ser Leu Asp Asp
        435                 440                 445

Leu Ile Asp Cys Cys Lys Thr Asp Ile Ile Ser Glu Phe Glu Val Gly
    450                 455                 460

Val Phe Thr Gly Asn Tyr Val Thr Gly Val Glu Asp Val Tyr Leu Gln
465                 470                 475                 480

Glu Leu Glu Arg Cys Arg Ala Leu Asn Asn Ser Asn Lys Gly Glu Ala
                485                 490                 495

Lys Ala Glu Val Asp Ile Gly Leu Tyr Asn Ser Ala Asp Tyr
            500                 505                 510

(2) INFORMATION ZU SEQ ID NO: 6:

(i) SEQUENZ CHARAKTERISTIKA:
(A) LÄNGE: 371 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 6:

Met Ser Ser Gly Asn Ile Trp Lys Gln Leu Leu Glu Glu Asn Ser Glu
1               5                   10                  15

Gln Leu Asp Gln Ser Thr Thr Glu Thr Tyr Val Val Cys Cys Glu Asn
            20                  25                  30

Glu Asp Ser Leu Asn Gln Phe Leu Gln Gln Cys Trp Gln Ile Asp Glu
        35                  40                  45

Gly Glu Lys Val Thr Asn Leu Glu Pro Leu Gly Phe Phe Thr Lys Val
    50                  55                  60

Val Ser Arg Asp Glu Glu Asn Leu Arg Leu Asn Val Tyr Tyr Ala Lys
65                  70                  75                  80

Ser Pro Leu Asp Ala Gln Thr Leu Gln Phe Leu Gly Val Phe Leu Arg
                85                  90                  95

Gln Met Glu Thr Ser Gln Ile Arg Trp Ile Phe Leu Leu Asp Trp Leu
            100                 105                 110

Leu Asp Asp Lys Arg Leu Trp Leu Arg Gln Leu Arg Asn Ser Trp Ala
        115                 120                 125

Ala Leu Glu Glu Ala Gln Val Ala Pro Phe Pro Gly Gly Ala Val Val
    130                 135                 140

Val Val Leu Asn Pro Ser His Val Thr Gln Leu Glu Arg Asn Thr Met
145                 150                 155                 160

Val Trp Asn Ser Arg Arg Leu Asp Leu Val His Gln Thr Leu Arg Ala
                165                 170                 175

Ala Cys Leu Asn Thr Gly Ser Ala Leu Val Thr Leu Asp Pro Asn Thr
            180                 185                 190

Ala Arg Glu Asp Val Met His Ile Cys Ala Leu Leu Ala Gly Leu Pro
        195                 200                 205

Thr Ser Arg Pro Val Ala Met Leu Ser Leu Gln Ser Leu Phe Ile Pro
    210                 215                 220

His Gly Ala Asp Ser Ile Gly Lys Ile Cys Thr Ile Ala Pro Glu Phe
225                 230                 235                 240

Pro Val Ala Thr Val Phe Asp Asn Asp Phe Val Ser Ser Thr Phe Glu
                245                 250                 255

Ala Ala Ile Ala Pro Glu Leu Thr Pro Gly Pro Arg Val Pro Ser Asp
            260                 265                 270

His Pro Trp Leu Thr Glu Pro Thr Asn Pro Pro Ser Glu Ala Thr Ala
        275                 280                 285

Trp His Phe Asp Leu Gln Gly Arg Leu Ala Thr Leu Tyr Arg His Leu
    290                 295                 300

Gly Asp Ser Asn Lys Ala Ile Ser Val Thr Gln His Arg Phe His Lys
305                 310                 315                 320

Pro Arg Ser Glu Asp Tyr Ala Tyr Glu Phe Glu Leu Pro Ser Lys His
                325                 330                 335

Pro Thr Ile Arg Asp Leu Ile Arg Ser Ala Ala Ala Asp Ser Pro Asn
            340                 345                 350

Asp Val Ala Asp Ser Ile Asp Gly Leu Met Asp Gly Ile Val Gln Arg
        355                 360                 365

Asn Val His
    370

(2) INFORMATION ZU SEQ ID NO: 7:

(i) SEQUENZ CHARAKTERISTIKA:
(A) LÄNGE: 3616 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: NEIN

(ix) MERKMALE:
(A) NAME/SCHLÜSSEL: 5'UTR
(B) LAGE: 1..863

(ix) MERKMALE:
(A) NAME/SCHLÜSSEL: CDS
(B) LAGE: 864..1316

(ix) MERKMALE:
(A) NAME/SCHLÜSSEL: intron
(B) LAGE: 1317..1477

(ix) MERKMALE:
(A) NAME/SCHLÜSSEL: CDS
(B) LAGE: 1478..2592

(ix) MERKMALE:
(A) NAME/SCHLÜSSEL: 3'UTR
(B) LAGE: 2593..3616

(xi) SEQUENZBESCHREIBUNG: SEQ ID NO: 7:

GGGCCCGGTG CCAGCTCGCC AGGTGCGGAC TCGCGCTCGG GCTGTGGGCG CTCTACCTGC 60

TGCTGCTCGG CAGCTGCCTG ACGCGCGCGT ACGAGCTGTC GGATCTCGAA AACCTGGAAT 120

CCGATTACTA CAGCTACGTG CTGGATGTGA ACTTCGCGCT GCTGAGCGCC ATGAGCGCGA 180

CCGGCCTCGC GATGGGCGCC GTGAGCGGCT CCCTCGGGAG CGCGCCGGTG CTCGCGCAGT 240

GGCCGGCAGC GATCTGGGCC GTGCGCTTCC TGCGCGCCGC GGGCTATGTC GCGATAGTCC 300

TAATCCTGCC GTTCCTGTCC GTCGTCGCAT TCCTGCAGCC GCTCTGCGAG CGCGCGCTGG 360

CGCTGTTCCC GTTTGTGCGC GCGTGGGGCA TGGACGGCGT GTTCAACTTC CTGCTGCTCT 420

CCGCCGTGCT CTGGACTGTA TTCCTGGCCG TTCGCCTGCT CCGCGCCGTC TACAGACTGC 480

TGCGCTGGCT GGTCGGTCTT TTGGTCCGCC TGGCACGCCT GCTGCTGCGA GGCGCCCGTC 540

GGACGCCTGC GGCGGCCCCC GAGGAGCCCG TCTAGCGTGC GCGCGTTCTA GGCCCCTGAC 600

AGCTCCTACC TGGTGCTGGC CGCCGGTAGG GCTCGCATCG TGCGGCGCAG GCCCATTGCT 660

TTTTGGCCCC CGCTGGATCA TCGTTTCTTT TACGTGAAAA GTTTGCAGCG ATGAGCTGCA 720

GTATAAATAG GTTTTCTAGA TGCGCCAAAT CCCAGCTGGG TTTACCGGCG TCTGTTCGGG 780

ATAGTTACTT GATGGATGGG TCAACTTGAG AGCTTGGGTT TAGTGTTGAC TCCTTCTCTT 840

CATAGCACGC CGAACAAAGC GCA ATG ACT TAC AGA GAC GCA GCC ACG GCA 890
Met Thr Tyr Arg Asp Ala Ala Thr Ala
1               5

CTG GAG CAC CTG GCG ACG TAC GCC GAG AAG GAC GGG CTG TCC GTG GAG 938
Leu Glu His Leu Ala Thr Tyr Ala Glu Lys Asp Gly Leu Ser Val Glu
10                  15                  20                  25

CAG TTG ATG GAC TCC AAG ACG CGG GGC GGG TTG ACG TAC AAC GAC TTC 986
Gln Leu Met Asp Ser Lys Thr Arg Gly Gly Leu Thr Tyr Asn Asp Phe
                30                  35                  40

CTG GTC TTG CCG GGC AAG ATC GAC TTC CCA TCG TCG GAG GTG GTG CTG 1034
Leu Val Leu Pro Gly Lys Ile Asp Phe Pro Ser Ser Glu Val Val Leu
            45                  50                  55

TCG TCG CGC CTG ACC AAG AAG ATC ACC TTG AAC GCG CCG TTT GTG TCG 1082
Ser Ser Arg Leu Thr Lys Lys Ile Thr Leu Asn Ala Pro Phe Val Ser
        60                  65                  70

TCG CCG ATG GAC ACG GTG ACG GAG GCC GAC ATG GCG ATC CAC ATG GCG 1130
Ser Pro Met Asp Thr Val Thr Glu Ala Asp Met Ala Ile His Met Ala
    75                  80                  85

CTC CTG GGC GGC ATC GGG ATC ATC CAC CAC AAC TGC ACT GCG GAG GAG 1178
Leu Leu Gly Gly Ile Gly Ile Ile His His Asn Cys Thr Ala Glu Glu
90                  95                  100                 105

CAG GCG GAG ATG GTG CGC CGG GTC AAG AAG TAC GAA AAC GGG TTC ATC 1226
Gln Ala Glu Met Val Arg Arg Val Lys Lys Tyr Glu Asn Gly Phe Ile
                110                 115                 120

AAC GCC CCC GTG GTC GTG GGG CCG GAC GCG ACG GTG GCG GAC GTG CGC 1274
Asn Ala Pro Val Val Val Gly Pro Asp Ala Thr Val Ala Asp Val Arg
            125                 130                 135

CGG ATG AAG AAC GAG TTT GGG TTT GCA GGA TTT CCT GTG ACA 1316
Arg Met Lys Asn Glu Phe Gly Phe Ala Gly Phe Pro Val Thr
        140                 145                 150

GGTATGTTAG AGTGGCACGC GGGGCTGCAC GCTGGGATGA TGATCATAAA TCAATAACTT 1376

TCGTTCTACT GACTGCGATC AAACGATCGT GTAGACACCT TTTACTCTGA CCGCAGACGT 1436

GCAGCGCCTT TTTGGCAGGA ACATGTACTA ACACATCAGC A GAT GAT GGC AAG 1489
Asp Asp Gly Lys
1

CCG ACC GGG AAG CTG CAG GGG ATC ATC ACG TCC CGT GAC ATC CAG TTT 1537
Pro Thr Gly Lys Leu Gln Gly Ile Ile Thr Ser Arg Asp Ile Gln Phe
5 10 15 20

GTC GAG GAC GAG ACC CTG CTT GTG TCT GAG ATC ATG ACC AAG GAC GTC 1585
Val Glu Asp Glu Thr Leu Leu Val Ser Glu Ile Met Thr Lys Asp Val
25 30 35

ATC ACT GGG AAG CAG GGC ATC AAC CTC GAG GAG GCG AAC CAG ATC CTG 1633
Ile Thr Gly Lys Gln Gly Ile Asn Leu Glu Glu Ala Asn Gln Ile Leu
40 45 50

AAG AAC ACC AAG AAG GGC AAG CTG CCA ATT GTG GAC GAG GCG GGC TGC 1681
Lys Asn Thr Lys Lys Gly Lys Leu Pro Ile Val Asp Glu Ala Gly Cys
55 60 65

CTG GTG TCC ATG CTT TCG AGA ACT GAC TTG ATG AAG AAC CAG TCC TAC 1729
Leu Val Ser Met Leu Ser Arg Thr Asp Leu Met Lys Asn Gln Ser Tyr
70 75 80

CCA TTG GCC TCC AAG TCT GCC GAC ACC AAG CAG CTG CTC TGT GGT GCT 1777
Pro Leu Ala Ser Lys Ser Ala Asp Thr Lys Gln Leu Leu Cys Gly Ala
85 90 95 100

GCG ATC GGC ACC ATC GAC GCG GAC AGG CAG AGA CTG GCG ATG CTG GTC 1825
Ala Ile Gly Thr Ile Asp Ala Asp Arg Gln Arg Leu Ala Met Leu Val
105 110 115

GAG GCC GGT CTG GAC GTT GTT GTG CTA GAC TCC TCG CAG GGT AAC TCG 1873
Glu Ala Gly Leu Asp Val Val Val Leu Asp Ser Ser Gln Gly Asn Ser
120 125 130

GTC TTC CAG ATC AAC ATG ATC AAG TGG ATC AAG GAG ACC TTC CCA GAC 1921
Val Phe Gln Ile Asn Met Ile Lys Trp Ile Lys Glu Thr Phe Pro Asp
135 140 145

CTG CAG GTC ATT GCT GGC AAC GTG GTC ACC AGA GAG CAG GCT GCC AGC 1969
Leu Gln Val Ile Ala Gly Asn Val Val Thr Arg Glu Gln Ala Ala Ser
150 155 160

TTG ATC CAC GCC GGC GCA GAC GGG TTG CGT ATC GGT ATG GGC TCT GGC 2017
Leu Ile His Ala Gly Ala Asp Gly Leu Arg Ile Gly Met Gly Ser Gly
165 170 175 180

TCC ATC TGT ATC ACT CAG GAG GTG ATG GCC TGT GGT AGA CCA CAG GGT 2065
Ser Ile Cys Ile Thr Gln Glu Val Met Ala Cys Gly Arg Pro Gln Gly
185 190 195

ACC GCT GTC TAC AAC GTC ACG CAG TTC GCC AAC CAG TTT GGT GTG CCA 2113
Thr Ala Val Tyr Asn Val Thr Gln Phe Ala Asn Gln Phe Gly Val Pro
200 205 210

TGT ATT GCT GAC GGT GGT GTC CAG AAC ATC GGG CAC ATT ACC AAA GCT 2161
Cys Ile Ala Asp Gly Gly Val Gln Asn Ile Gly His Ile Thr Lys Ala
215 220 225

ATC GCT CTT GGC GCG TCC ACC GTC ATG ATG GGC GGT ATG CTG GCA GGC 2209
Ile Ala Leu Gly Ala Ser Thr Val Met Met Gly Gly Met Leu Ala Gly
230 235 240

ACT ACA GAG TCT CCA GGC GAG TAC TTC TTC AGG GAC GGG AAG AGA CTG 2257
Thr Thr Glu Ser Pro Gly Glu Tyr Phe Phe Arg Asp Gly Lys Arg Leu
245 250 255 260

AAG ACC TAC AGA GGT ATG GGC TCC ATC GAC GCC ATG CAA AAG ACT GAT 2305
Lys Thr Tyr Arg Gly Met Gly Ser Ile Asp Ala Met Gln Lys Thr Asp
265 270 275

GTC AAG GGT AAC GCC GCT ACC TCC CGT TAC TTC TCT GAG TCT GAC AAG 2353
Val Lys Gly Asn Ala Ala Thr Ser Arg Tyr Phe Ser Glu Ser Asp Lys
280 285 290

GTT CTG GTC GCT CAG GGT GTT ACT GGT TCT GTG ATC GAC AAG GGC TCC 2401
Val Leu Val Ala Gln Gly Val Thr Gly Ser Val Ile Asp Lys Gly Ser
295 300 305

ATC AAG AAG TAC ATT CCA TAT CTG TAC AAT GGT CTA CAG CAC TCG TGC 2449
Ile Lys Lys Tyr Ile Pro Tyr Leu Tyr Asn Gly Leu Gln His Ser Cys
310 315 320

CAG GAT ATC GGT GTG CGC TCT CTA GTG GAG TTC AGA GAG AAG GTG GAC 2497
Gln Asp Ile Gly Val Arg Ser Leu Val Glu Phe Arg Glu Lys Val Asp
325 330 335 340

TCT GGC TCG GTC AGA TTT GAG TTC AGA ACT CCA TCT GCC CAG TTG GAG 2545
Ser Gly Ser Val Arg Phe Glu Phe Arg Thr Pro Ser Ala Gln Leu Glu
345 350 355

GGT GGT GTG CAC AAC TTG CAC TCC TAC GAG AAG CGC CTA TTT GACTGAGTGC 2597
Gly Gly Val His Asn Leu His Ser Tyr Glu Lys Arg Leu Phe Asp
360 365 370

CACTAGGCCC ACACTATAGA AGTGGATCCG GGCGCGATGG CACCCATACT TTTATATTAT 2657

GTTGATTGAT GTACGTAAAC GATAGATATA ATAACAGACG CGGCATCTCA TTTGTATGCA 2717

ATATATCTGG AACATGGTTA TGCGTACTCA ACTGTATGTA CTACTTTATA TACACAGCTC 2777

TGGGACACTT GGTGAGATAT ATGTTTCATT ATGTATGCCT CGCTATCGAA AGGTCTGGCA 2837

TTATGGGCTA CTGGGTCTAA GAGTCATGGC TTATGAGTAT TTATTTATTT ATTTCTCTTC 2897

CTTTTCATTA AACTCCTCGA GCTTCTTTCT GTAATACTGC TCTCTAGACT TCTCCACATC 2957

TGCTAATGAT GGTGGAAGTC GTTCGTTTTC CAAATCCGCT CTACGAGCGC GCTCGAAGTT 3017

AGACAGCGCC TCGTTCAGAC CTTCAGACCC GCGTGACAGC GCTCCACGAG GCAGCACGCC 3077

AGAATTCATT GTTTTTAGGT ACTGCACCTT ATCGCTCTCT TCTCTCAACA CGCTATACAT 3137

TCGGGAAACC TTGGCAATCG CCAATATTTT ACTGCGTAGT GCACGCCGTT TTGCATCATC 3197

GTCCAGAATA GACCGTTTTT TCTTCGATTT CTTGGAGCCA GGTATAACAG TTACAACCTG 3257

CTCAGTGTTT TTGGACTTCA ATGTAGCACC TAAGTCCTCC CTTATAACAA AAGTCTCTTC 3317

CTCCAATTCT TCTTCAGTAC AAATGTTTAA TATCGAAACC AACATTTCAG TCACTTTCTC 3377

GCCAACAAAT GGCAAAGACC AGGTGAATAC GTCCATGAAA TTCGGTAACC AATACGGATG 3437

CTGTGACATG TTAAATTGTC TAATGTTCAT AACGTTATCC GAGTATTTTA GGACCGCGGC 3497

CTTGTTCTTG TAAGTGTCCA AGTAGTTGGG TGCGCTGAAC AACGTAAGTA AACTAGGAAA 3557

GCCCAGATTC TTGGTATTCT TGTACATTCT GTAGCCCTGA TCTTGGGCTT CGTGGGCCC 3616

(2) INFORMATION ZU SEQ ID NO: 8:

(i) SEQUENZ CHARAKTERISTIKA:
(A) LÄNGE: 151 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 8:

Met Thr Tyr Arg Asp Ala Ala Thr Ala Leu Glu His Leu Ala Thr Tyr
1               5                   10                  15

Ala Glu Lys Asp Gly Leu Ser Val Glu Gln Leu Met Asp Ser Lys Thr
            20                  25                  30

Arg Gly Gly Leu Thr Tyr Asn Asp Phe Leu Val Leu Pro Gly Lys Ile
        35                  40                  45

Asp Phe Pro Ser Ser Glu Val Val Leu Ser Ser Arg Leu Thr Lys Lys
    50                  55                  60

Ile Thr Leu Asn Ala Pro Phe Val Ser Ser Pro Met Asp Thr Val Thr
65                  70                  75                  80

Glu Ala Asp Met Ala Ile His Met Ala Leu Leu Gly Gly Ile Gly Ile
                85                  90                  95

Ile His His Asn Cys Thr Ala Glu Glu Gln Ala Glu Met Val Arg Arg
            100                 105                 110

Val Lys Lys Tyr Glu Asn Gly Phe Ile Asn Ala Pro Val Val Val Gly
        115                 120                 125

Pro Asp Ala Thr Val Ala Asp Val Arg Arg Met Lys Asn Glu Phe Gly
    130                 135                 140

Phe Ala Gly Phe Pro Val Thr
145                 150

(2) INFORMATION ZU SEQ ID NO: 9:

    (i) SEQUENZ CHARAKTERISTIKA:
        (A) LÄNGE: 371 Aminosäuren
        (B) ART: Aminosäure
        (D) TOPOLOGIE: linear

    (ii) ART DES MOLEKÜLS: Protein

    (xi) SEQUENZBESCHREIBUNG: SEQ ID NO: 9:

Asp Asp Gly Lys Pro Thr Gly Lys Leu Gln Gly Ile Ile Thr Ser Arg
  1               5                  10                  15

Asp Ile Gln Phe Val Glu Asp Glu Thr Leu Leu Val Ser Glu Ile Met
             20                  25                  30

Thr Lys Asp Val Ile Thr Gly Lys Gln Gly Ile Asn Leu Glu Glu Ala
         35                  40                  45

Asn Gln Ile Leu Lys Asn Thr Lys Lys Gly Lys Leu Pro Ile Val Asp
     50                  55                  60

Glu Ala Gly Cys Leu Val Ser Met Leu Ser Arg Thr Asp Leu Met Lys
 65                  70                  75                  80

Asn Gln Ser Tyr Pro Leu Ala Ser Lys Ser Ala Asp Thr Lys Gln Leu
                 85                  90                  95

Leu Cys Gly Ala Ala Ile Gly Thr Ile Asp Ala Asp Arg Gln Arg Leu
            100                 105                 110

Ala Met Leu Val Glu Ala Gly Leu Asp Val Val Val Leu Asp Ser Ser
        115                 120                 125

Gln Gly Asn Ser Val Phe Gln Ile Asn Met Ile Lys Trp Ile Lys Glu
    130                 135                 140

Thr Phe Pro Asp Leu Gln Val Ile Ala Gly Asn Val Val Thr Arg Glu
145                 150                 155                 160

Gln Ala Ala Ser Leu Ile His Ala Gly Ala Asp Gly Leu Arg Ile Gly
                165                 170                 175

Met Gly Ser Gly Ser Ile Cys Ile Thr Gln Glu Val Met Ala Cys Gly
            180                 185                 190

Arg Pro Gln Gly Thr Ala Val Tyr Asn Val Thr Gln Phe Ala Asn Gln
        195                 200                 205

Phe Gly Val Pro Cys Ile Ala Asp Gly Gly Val Gln Asn Ile Gly His
    210                 215                 220

Ile Thr Lys Ala Ile Ala Leu Gly Ala Ser Thr Val Met Met Gly Gly
225                 230                 235                 240

Met Leu Ala Gly Thr Thr Glu Ser Pro Gly Glu Tyr Phe Phe Arg Asp
                245                 250                 255

Gly Lys Arg Leu Lys Thr Tyr Arg Gly Met Gly Ser Ile Asp Ala Met
            260                 265                 270

Gln Lys Thr Asp Val Lys Gly Asn Ala Ala Thr Ser Arg Tyr Phe Ser
        275                 280                 285

Glu Ser Asp Lys Val Leu Val Ala Gln Gly Val Thr Gly Ser Val Ile
    290                 295                 300

Asp Lys Gly Ser Ile Lys Lys Tyr Ile Pro Tyr Leu Tyr Asn Gly Leu
305                 310                 315                 320

Gln His Ser Cys Gln Asp Ile Gly Val Arg Ser Leu Val Glu Phe Arg
                325                 330                 335

Glu Lys Val Asp Ser Gly Ser Val Arg Phe Glu Phe Arg Thr Pro Ser
            340                 345                 350

Ala Gln Leu Glu Gly Gly Val His Asn Leu His Ser Tyr Glu Lys Arg
        355                 360                 365

Leu Phe Asp
    370

(2) INFORMATION ZU SEQ ID NO: 10:

(i) SEQUENZ CHARAKTERISTIKA:
(A) LÄNGE: 2697 Basenpaare
(B) ART: Nukleinsäure
(C) STRANGFORM: Einzel
(D) TOPOLOGIE: linear

(ii) ART DES MOLEKÜLS: DNS (genomisch)

(iii) HYPOTHETISCH: NEIN

(iii) ANTISENSE: NEIN

(ix) MERKMALE:
(A) NAME/SCHLÜSSEL: 5'UTR
(B) LAGE: 1..455

(ix) MERKMALE:
(A) NAME/SCHLÜSSEL: CDS
(B) LAGE: 456..2033

(ix) MERKMALE:
(A) NAME/SCHLÜSSEL: 3'UTR
(B) LAGE: 2034..2697

(xi) SEQUENZBESCHREIBUNG: SEQ ID NO: 10:

ATCGATTTCA GGAGATTTTT GGTAGCATTA TTGAGGTCAT TAGAGGCGTT CTGTGACTTT 60

CGACGATTTG CACGCGCAGA AGAGGGCGTT CAACCAGCCT TTCGGATATT CCGGTTCGAG 120

TTATACCAGC AGGGATCAGC GCAGGCACTA GAGTGGCGGG TGCTAATAAG AGGAGCAGGT 180

CCTGGAACTG AAGTTGCAAG AGATAAGCAT TGCGCGGAGA AGGAGGCGGT TAGAGGGTGC 240

AAGCGAGCAG GATGGGGTCT TCGATGAACT TCCCGTCTGG GTATGTGAAC AAGCACACGC 300

TGCAGGCACA CCGGTAGGGC GAGTGCAGGG TGAAAAATAT ATATGCGCTC GAGAAGCGCT 360

GGGGATGAGT TCGTCTGCAA CGGCAGGCGG ATCTTCATCT GACAAAACCA GCTGCCTACA 420

TCAGTGCGAA GCTGTTCAGT GATAGAATAG GAGTA ATG GCT GCT GTT GAA CAA 473
Met Ala Ala Val Glu Gln
1               5

GTT TCT AGC GTG TTT GAC ACC ATT TTG GTG CTG GAC TTC GGG TCC CAG 521
Val Ser Ser Val Phe Asp Thr Ile Leu Val Leu Asp Phe Gly Ser Gln
            10                  15                  20

TAC TCG CAT CTG ATC ACG CGG CGG CTG CGT GAG TTT AAT GTG TAC GCG 569
Tyr Ser His Leu Ile Thr Arg Arg Leu Arg Glu Phe Asn Val Tyr Ala
        25                  30                  35

GAG ATG CTT CCG TGT ACG CAG AAG ATC AGC GAG CTG GGC TGG AAG CCA 617
Glu Met Leu Pro Cys Thr Gln Lys Ile Ser Glu Leu Gly Trp Lys Pro
    40                  45                  50

AAG GGT GTG ATT TTG TCA GGC GGG CCG TAC TCC GTG TAC GCG GCA GAT 665
Lys Gly Val Ile Leu Ser Gly Gly Pro Tyr Ser Val Tyr Ala Ala Asp
55                  60                  65                  70

GCT CCG CAC GTG GAC CGG GCG GTG TTC GAG TTG GGC GTT CCA ATT CTG 713
Ala Pro His Val Asp Arg Ala Val Phe Glu Leu Gly Val Pro Ile Leu
                75                  80                  85

GGC ATC TGC TAC GGG CTA CAG GAG CTT GCG TGG ATA GCC GGC GCA GAG 761
Gly Ile Cys Tyr Gly Leu Gln Glu Leu Ala Trp Ile Ala Gly Ala Glu
            90                  95                  100

GTG GGG CGC GGC GAG AAG CGC GAG TAC GGG CGC GCG ACG CTG CAC GTG 809
Val Gly Arg Gly Glu Lys Arg Glu Tyr Gly Arg Ala Thr Leu His Val
        105                 110                 115

GAG GAC AGC GCG TGC CCG CTG TTC AAC AAC GTG GAC AGC AGC ACG GTG 857
Glu Asp Ser Ala Cys Pro Leu Phe Asn Asn Val Asp Ser Ser Thr Val
    120                 125                 130

TGG ATG TCG CAC GGT GAC AAG CTG CAC GCA CTA CCT GCG GAT TTC CAC 905
Trp Met Ser His Gly Asp Lys Leu His Ala Leu Pro Ala Asp Phe His
135                 140                 145                 150

GTC ACT GCG ACG ACG GAG AAC TCT CCT TTC TGC GGG ATT GCA CAC GAC 953
Val Thr Ala Thr Thr Glu Asn Ser Pro Phe Cys Gly Ile Ala His Asp
                155                 160                 165

TCG AAG CCA ATC TTC GGG ATC CAG TTC CAC CCT GAG GTG ACG CAC TCC 1001
Ser Lys Pro Ile Phe Gly Ile Gln Phe His Pro Glu Val Thr His Ser
            170                 175                 180

TCG CAG GGG AAG ACG TTG CTG AAG AAC TTT GCG GTG GAG ATC TGC CAG 1049
Ser Gln Gly Lys Thr Leu Leu Lys Asn Phe Ala Val Glu Ile Cys Gln
        185                 190                 195

GCC GCG CAG ACC TGG ACG ATG GAA AAC TTC ATT GAC ACC GAG ATC CAG 1097
Ala Ala Gln Thr Trp Thr Met Glu Asn Phe Ile Asp Thr Glu Ile Gln
    200                 205                 210

CGG ATC CGG ACC CTT GTG GGC CCC ACC GCG GAA GTC ATC GGT GCT GTG 1145
Arg Ile Arg Thr Leu Val Gly Pro Thr Ala Glu Val Ile Gly Ala Val
215                 220                 225                 230

TCC GGC GGT GTC GAC TCG ACC GTC GCT GCG AAG CTG ATG ACC GAG GCC 1193
Ser Gly Gly Val Asp Ser Thr Val Ala Ala Lys Leu Met Thr Glu Ala
                235                 240                 245

ATC GGC GAC CGG TTC CAC GCG ATC CTG GTC GAC AAC GGT GTT CTG CGC 1241
Ile Gly Asp Arg Phe His Ala Ile Leu Val Asp Asn Gly Val Leu Arg
            250                 255                 260

CTC AAC GAA GCG GCC AAT GTG AAG AAA ATC CTC GGC GAG GGC TTG GGC 1289
Leu Asn Glu Ala Ala Asn Val Lys Lys Ile Leu Gly Glu Gly Leu Gly
        265                 270                 275

ATC AAC TTG ACT GTT GTT GAC GCC TCC GAA GAG TTC TTG ACG AAG CTC 1337
Ile Asn Leu Thr Val Val Asp Ala Ser Glu Glu Phe Leu Thr Lys Leu
    280                 285                 290

AAG GGC GTC ACG GAC CCT GAG AAG AAG AGA AAG ATC ATC GGT AAC ACC 1385
Lys Gly Val Thr Asp Pro Glu Lys Lys Arg Lys Ile Ile Gly Asn Thr
295                 300                 305                 310

TTC ATT CAT GTT TTT GAG CGC GAG GCA GCC AGG ATC CAG CCT AAG AAC 1433
Phe Ile His Val Phe Glu Arg Glu Ala Ala Arg Ile Gln Pro Lys Asn
                315                 320                 325

GGC GAG GAG ATT GAG TTC CTG TTG CAG GGT ACC CTA TAC CCT GAC GTT 1481
Gly Glu Glu Ile Glu Phe Leu Leu Gln Gly Thr Leu Tyr Pro Asp Val
            330                 335                 340

ATC GAG TCC ATT TCC TTT AAG GGC CCA TCT CAG ACG ATC AAG ACC CAC 1529
Ile Glu Ser Ile Ser Phe Lys Gly Pro Ser Gln Thr Ile Lys Thr His
        345                 350                 355

CAT AAC GTC GGT GGT CTT TTG GAC AAC ATG AAA CTG AAG CTC ATT GAG 1577
His Asn Val Gly Gly Leu Leu Asp Asn Met Lys Leu Lys Leu Ile Glu
    360                 365                 370

CCT TTG CGC GAG CTT TTC AAG GAC GAG GTG AGA CAC CTG GGA GAA CTA 1625
Pro Leu Arg Glu Leu Phe Lys Asp Glu Val Arg His Leu Gly Glu Leu
375                 380                 385                 390

TTG GGG ATC TCC CAC GAG TTG GTC TGG AGA CAT CCG TTC CCA GGC CCA 1673
Leu Gly Ile Ser His Glu Leu Val Trp Arg His Pro Phe Pro Gly Pro
                395                 400                 405

GGT ATC GCC ATC CGT GTG CTA GGC GAG GTC ACC AAG GAG CAG GTG GAG 1721
Gly Ile Ala Ile Arg Val Leu Gly Glu Val Thr Lys Glu Gln Val Glu
            410                 415                 420

ATT GCC AGA AAG GCA GAC CAC ATC TAC ATC GAG GAG ATC AGG AAA GCA 1769
Ile Ala Arg Lys Ala Asp His Ile Tyr Ile Glu Glu Ile Arg Lys Ala
        425                 430                 435

GGT CTA TAC AAC AAG ATT TCT CAA GCT TTT GCT TGC TTG CTG CCT GTT 1817
Gly Leu Tyr Asn Lys Ile Ser Gln Ala Phe Ala Cys Leu Leu Pro Val
    440                 445                 450

AAG TCT GTG GGT GTC ATG GGT GAC CAG AGA ACC TAC GAC CAG GTC ATT 1865
Lys Ser Val Gly Val Met Gly Asp Gln Arg Thr Tyr Asp Gln Val Ile
455                 460                 465                 470

GCT CTA AGA GCA ATT GAG ACC ACG GAC TTC ATG ACT GCC GAC TGG TAT 1913
Ala Leu Arg Ala Ile Glu Thr Thr Asp Phe Met Thr Ala Asp Trp Tyr
                475                 480                 485

CCA TTT GAG CAC GAA TTC TTG AAG CAT GTC GCA TCC CGT ATT GTT AAC 1961
Pro Phe Glu His Glu Phe Leu Lys His Val Ala Ser Arg Ile Val Asn
            490                 495                 500

GAG GTT GAA GGT GTT GCC AGA GTC ACC TAC GAC ATA ACT TCT AAG CCT 2009
Glu Val Glu Gly Val Ala Arg Val Thr Tyr Asp Ile Thr Ser Lys Pro
        505                 510                 515

CCA GCT ACC GTT GAA TGG GAA TAATCACCCT TGGGATCCGC TGACTGGCTA 2060
Pro Ala Thr Val Glu Trp Glu
    520                 525

CTGTAATTCT ATGTAGTGGA TTAGTACGAT AAGTTACTTT TGTATGATAG ATGTAATCAC 2120

ATCTGGCTAT TAAAATGACT CAGCCGAGGT AAATCTAACG TCCCTTCACA AGGGTGTTCC 2180

TGTGTGGACT TCCGCCTGAA TTTTTATAGA TATATAGATA CTCTACTCAT GAACAACCTG 2240

CAACCGAATA AGCATTAGTG CCAGGAGAAG AGAACCGTGG AAATGGGGCA AGTAGAAAAA 2300

ATCATATTCC TTAAGAATAA GACAGTACCA GAGGACCATT ACGAGACGAT TTTTGAATCG 2360

AATGGCTTCC AGACTCACTT TGTACCCATA ATAACCCATG AACACCTGCC AGATGAGGTT 2420

CGCGGTCGAC TATCCGACGC GAATTACATG AAAAGGTTGA ATTGTTTGGT GGTAACCTCT 2480

CAGAGGACTG TGGAGTGTCT CTATGAGGAC GTTCTGCCCT CTCTTCCAGC TGAAGCACGC 2540

AAATCTCTTC TCAATACGCC AGTATTCGTG GTTGGGCGTG CCACTCAGGA ATTTATGGAG 2600

AGATGCGGCT TTACGGACGT GAGAGGGGGA TCTGAGACTG GTAATGGCGT TTTGCTAGCG 2660

GAGTTAATGT TAAATATGAT CCAGAAGGGC GATGGGG 2697

(2) INFORMATION ZU SEQ ID NO: 11:

(i) SEQUENZ CHARAKTERISTIKA:
(A) LÄNGE: 525 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 11:

Met Ala Ala Val Glu Gln Val Ser Ser Val Phe Asp Thr Ile Leu Val
1               5                   10                  15

Leu Asp Phe Gly Ser Gln Tyr Ser His Leu Ile Thr Arg Arg Leu Arg
            20                  25                  30

Glu Phe Asn Val Tyr Ala Glu Met Leu Pro Cys Thr Gln Lys Ile Ser
        35                  40                  45

Glu Leu Gly Trp Lys Pro Lys Gly Val Ile Leu Ser Gly Gly Pro Tyr
    50                  55                  60

Ser Val Tyr Ala Ala Asp Ala Pro His Val Asp Arg Ala Val Phe Glu
65                  70                  75                  80

Leu Gly Val Pro Ile Leu Gly Ile Cys Tyr Gly Leu Gln Glu Leu Ala
                85                  90                  95

Trp Ile Ala Gly Ala Glu Val Gly Arg Gly Glu Lys Arg Glu Tyr Gly
            100                 105                 110

Arg Ala Thr Leu His Val Glu Asp Ser Ala Cys Pro Leu Phe Asn Asn
        115                 120                 125

Val Asp Ser Ser Thr Val Trp Met Ser His Gly Asp Lys Leu His Ala
    130                 135                 140

Leu Pro Ala Asp Phe His Val Thr Ala Thr Thr Glu Asn Ser Pro Phe
145                 150                 155                 160

Cys Gly Ile Ala His Asp Ser Lys Pro Ile Phe Gly Ile Gln Phe His
                165                 170                 175

Pro Glu Val Thr His Ser Ser Gln Gly Lys Thr Leu Leu Lys Asn Phe
            180                 185                 190

Ala Val Glu Ile Cys Gln Ala Ala Gln Thr Trp Thr Met Glu Asn Phe
        195                 200                 205

Ile Asp Thr Glu Ile Gln Arg Ile Arg Thr Leu Val Gly Pro Thr Ala
    210                 215                 220

Glu Val Ile Gly Ala Val Ser Gly Gly Val Asp Ser Thr Val Ala Ala
225                 230                 235                 240

Lys Leu Met Thr Glu Ala Ile Gly Asp Arg Phe His Ala Ile Leu Val
                245                 250                 255

Asp Asn Gly Val Leu Arg Leu Asn Glu Ala Ala Asn Val Lys Lys Ile
            260                 265                 270

Leu Gly Glu Gly Leu Gly Ile Asn Leu Thr Val Val Asp Ala Ser Glu
        275                 280                 285

Glu Phe Leu Thr Lys Leu Lys Gly Val Thr Asp Pro Glu Lys Lys Arg
    290                 295                 300

Lys Ile Ile Gly Asn Thr Phe Ile His Val Phe Glu Arg Glu Ala Ala
305                 310                 315                 320

Arg Ile Gln Pro Lys Asn Gly Glu Glu Ile Glu Phe Leu Leu Gln Gly
                325                 330                 335

Thr Leu Tyr Pro Asp Val Ile Glu Ser Ile Ser Phe Lys Gly Pro Ser
            340                 345                 350

Gln Thr Ile Lys Thr His His Asn Val Gly Gly Leu Leu Asp Asn Met
        355                 360                 365

Lys Leu Lys Leu Ile Glu Pro Leu Arg Glu Leu Phe Lys Asp Glu Val
    370                 375                 380

Arg His Leu Gly Glu Leu Leu Gly Ile Ser His Glu Leu Val Trp Arg
385                 390                 395                 400

His Pro Phe Pro Gly Pro Gly Ile Ala Ile Arg Val Leu Gly Glu Val
                405                 410                 415

Thr Lys Glu Gln Val Glu Ile Ala Arg Lys Ala Asp His Ile Tyr Ile
            420                 425                 430

Glu Glu Ile Arg Lys Ala Gly Leu Tyr Asn Lys Ile Ser Gln Ala Phe
        435                 440                 445

Ala Cys Leu Leu Pro Val Lys Ser Val Gly Val Met Gly Asp Gln Arg
    450                 455                 460

Thr Tyr Asp Gln Val Ile Ala Leu Arg Ala Ile Glu Thr Thr Asp Phe
465                 470                 475                 480

Met Thr Ala Asp Trp Tyr Pro Phe Glu His Glu Phe Leu Lys His Val
                485                 490                 495

Ala Ser Arg Ile Val Asn Glu Val Glu Gly Val Ala Arg Val Thr Tyr
            500                 505                 510

Asp Ile Thr Ser Lys Pro Pro Ala Thr Val Glu Trp Glu
        515                 520                 525

(2) INFORMATION ZU SEQ ID NO: 12:

(i) SEQUENZ CHARAKTERISTIKA:
(A) LÄNGE: 1634 Basenpaare
(B) ART: Nukleinsäure
(C) STRANGFORM: Doppel
(D) TOPOLOGIE: linear

(ii) ART DES MOLEKÜLS: cDNS zu mRNS

(iii) HYPOTHETISCH: NEIN

(iii) ANTISENSE: NEIN

(ix) MERKMALE:
(A) NAME/SCHLÜSSEL: 5'UTR
(B) LAGE: 1..519

(ix) MERKMALE:
(A) NAME/SCHLÜSSEL: CDS
(B) LAGE: 520..1482

(ix) MERKMALE:
(A) NAME/SCHLÜSSEL: 3'UTR
(B) LAGE: 1483..1634

(xi) SEQUENZBESCHREIBUNG: SEQ ID NO: 12:

CCTCGAACAT CTATCTTCTG AGCTCGATAG TCTACGAAAT CGGCACACTA GCCTAATTGC 60

CGAGATGAAG AGCTCCAGGG AACCGTTAAA GATCTGATGT TCCATCTTCA ATCAGGACAA 120

ATGTTACGGG ATGTCCCTGA CGCCACAGAA GGTAGCCTGG TGGTCCAGAC AGAAAAAGAG 180

CCTACACCAA AGAAGAAACA TAACAAGAAA AAGCCTCCGC ATCGTTTTGG TAAATCATAA 240

TAGGCACGAT GCGCATATAC CCTGACCATC ATAGCGGTTC CCCCCGCTAA CTGCTCCGAG 300

CGGGTAACCC CATGTCACAA AGTGACTCTG TCTCTTCGTG GTAGGTGATG TCAAATTTTC 360

ACGACTTCCC ACCCCGATGA GCATCCGTAT TCCTTTTCAT CTAAATTCTA ATAGATGGCT 420

TATGGATTCT TATTGGCGAC TTACAAGCCT ATGTAGTTGG CTTCCCTCAA GTGTTCGTAG 480

TCTACCACCT CACACCCGGT CTAACAGCTT ACGAGAATA ATG GCT ACT AAT GCA 534
Met Ala Thr Asn Ala
1               5

ATC AAG CTT CTT GCG CCA GAT ATC CAC AGG GGT CTG GCA GAG CTG GTC 582
Ile Lys Leu Leu Ala Pro Asp Ile His Arg Gly Leu Ala Glu Leu Val
                10                  15                  20

GCT AAA CGC CTA GGC TTA CGT CTG ACA GAC TGC AAG CTT AAG CGG GAT 630
Ala Lys Arg Leu Gly Leu Arg Leu Thr Asp Cys Lys Leu Lys Arg Asp
            25                  30                  35

TGT AAC GGG GAG GCG ACA TTT TCG ATC GGA GAA TCT GTT CGA GAC CAG 678
Cys Asn Gly Glu Ala Thr Phe Ser Ile Gly Glu Ser Val Arg Asp Gln
        40                  45                  50

GAT ATC TAC ATC ATC ACG CAG GTG GGG TCC GGG GAC GTG AAC GAC CGA 726
Asp Ile Tyr Ile Ile Thr Gln Val Gly Ser Gly Asp Val Asn Asp Arg
    55                  60                  65

GTG CTG GAG CTG CTC ATC ATG ATC AAC GCT AGC AAG ACG GCG TCT GCG 774
Val Leu Glu Leu Leu Ile Met Ile Asn Ala Ser Lys Thr Ala Ser Ala
70                  75                  80                  85

CGG CGA ATT ACG GCT GTG ATT CCA AAC TTC CCA TAC GCG CGG CAG GAC 822
Arg Arg Ile Thr Ala Val Ile Pro Asn Phe Pro Tyr Ala Arg Gln Asp
                90                  95                  100

CGG AAG GAT AAG TCA CGG GCG CCA ATT ACC GCG AAG CTC ATG GCG GAC 870
Arg Lys Asp Lys Ser Arg Ala Pro Ile Thr Ala Lys Leu Met Ala Asp
            105                 110                 115

ATG CTG ACT ACC GCG GGC TGC GAT CAT GTC ATC ACC ATG GAC TTA CAC 918
Met Leu Thr Thr Ala Gly Cys Asp His Val Ile Thr Met Asp Leu His
        120                 125                 130

GCT TCG CAA ATC CAG GGC TTC TTT GAT GTA CCA GTT GAC AAC CTT TAC 966
Ala Ser Gln Ile Gln Gly Phe Phe Asp Val Pro Val Asp Asn Leu Tyr
    135                 140                 145

GCA GAG CCT AGC GTG GTG AAG TAT ATC AAG GAG CAT ATT CCC CAC GAC 1014
Ala Glu Pro Ser Val Val Lys Tyr Ile Lys Glu His Ile Pro His Asp
150                 155                 160                 165

GAT GCC ATC ATC ATC TCG CCG GAT GCT GGT GGT GCC AAA CGT GCG TCG 1062
Asp Ala Ile Ile Ile Ser Pro Asp Ala Gly Gly Ala Lys Arg Ala Ser
                170                 175                 180

CTT CTA TCA GAT CGC CTA AAC TTG AAC TTT GCG CTG ATT CAT AAG GAA 1110
Leu Leu Ser Asp Arg Leu Asn Leu Asn Phe Ala Leu Ile His Lys Glu
            185                 190                 195

CGT GCA AAG GCA AAC GAA GTG TCC CGC ATG GTT CTG GTC GGC GAT GTT 1158
Arg Ala Lys Ala Asn Glu Val Ser Arg Met Val Leu Val Gly Asp Val
        200                 205                 210

ACC GAT AAA GTC TGC ATT ATC GTT GAC GAT ATG GCG GAT ACT TGT GGT 1206
Thr Asp Lys Val Cys Ile Ile Val Asp Asp Met Ala Asp Thr Cys Gly
    215                 220                 225

ACG CTG GCC AAG GCG GCA GAA GTG CTG CTA GAG CAC AAC GCG CGG TCT 1254
Thr Leu Ala Lys Ala Ala Glu Val Leu Leu Glu His Asn Ala Arg Ser
230                 235                 240                 245

GTG ATA GCC ATT GTT ACC CAC GGT ATC CTT TCA GGA AAG GCC ATT GAG 1302
Val Ile Ala Ile Val Thr His Gly Ile Leu Ser Gly Lys Ala Ile Glu
                250                 255                 260

AAC ATC AAC AAT TCG AAG CTT GAT AGG GTT GTG TGT ACC AAC ACC GTG 1350
Asn Ile Asn Asn Ser Lys Leu Asp Arg Val Val Cys Thr Asn Thr Val
            265                 270                 275

CCA TTC GAG GAG AAG ATG AAG TTA TGC CCG AAG TTA GAT GTA ATT GAT 1398
Pro Phe Glu Glu Lys Met Lys Leu Cys Pro Lys Leu Asp Val Ile Asp
        280                 285                 290

ATC TCG GCA GTT CTT GCG GAA TCC ATT CGC CGT CTA CAC AAT GGT GAA 1446
Ile Ser Ala Val Leu Ala Glu Ser Ile Arg Arg Leu His Asn Gly Glu
    295                 300                 305

AGT ATC TCC TAC CTC TTT AAA AAC AAC CCA CTA TGATTTTGCT TCTCGATGCT 1499
Ser Ile Ser Tyr Leu Phe Lys Asn Asn Pro Leu
310                 315                 320

GGCTTCTTGA GGGCCAATTT TGCCGTAGAG GTAGTATCCC TTCTTTTTAT ATTGACTATT 1559

TAACGAAGAC TATTTCTTCA TAAATGGACT TCGGCTTCAC TGTGAATCTC ACATGATATA 1619

GTTGTTTCAG AGACC 1634

(2) INFORMATION ZU SEQ ID NO: 13:

(i) SEQUENZ CHARAKTERISTIKA:
(A) LÄNGE: 320 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 13:

Met Ala Thr Asn Ala Ile Lys Leu Leu Ala Pro Asp Ile His Arg Gly
1               5                   10                  15

Leu Ala Glu Leu Val Ala Lys Arg Leu Gly Leu Arg Leu Thr Asp Cys
            20                  25                  30

Lys Leu Lys Arg Asp Cys Asn Gly Glu Ala Thr Phe Ser Ile Gly Glu
        35                  40                  45

Ser Val Arg Asp Gln Asp Ile Tyr Ile Ile Thr Gln Val Gly Ser Gly
    50                  55                  60

Asp Val Asn Asp Arg Val Leu Glu Leu Leu Ile Met Ile Asn Ala Ser
65                  70                  75                  80

Lys Thr Ala Ser Ala Arg Arg Ile Thr Ala Val Ile Pro Asn Phe Pro
                85                  90                  95

Tyr Ala Arg Gln Asp Arg Lys Asp Lys Ser Arg Ala Pro Ile Thr Ala
            100                 105                 110

Lys Leu Met Ala Asp Met Leu Thr Thr Ala Gly Cys Asp His Val Ile
        115                 120                 125

Thr Met Asp Leu His Ala Ser Gln Ile Gln Gly Phe Phe Asp Val Pro
    130                 135                 140

Val Asp Asn Leu Tyr Ala Glu Pro Ser Val Val Lys Tyr Ile Lys Glu
145                 150                 155                 160

His Ile Pro His Asp Asp Ala Ile Ile Ile Ser Pro Asp Ala Gly Gly
                165                 170                 175

Ala Lys Arg Ala Ser Leu Leu Ser Asp Arg Leu Asn Leu Asn Phe Ala
            180                 185                 190

Leu Ile His Lys Glu Arg Ala Lys Ala Asn Glu Val Ser Arg Met Val
        195                 200                 205

Leu Val Gly Asp Val Thr Asp Lys Val Cys Ile Ile Val Asp Asp Met
    210                 215                 220

Ala Asp Thr Cys Gly Thr Leu Ala Lys Ala Ala Glu Val Leu Leu Glu
225                 230                 235                 240

His Asn Ala Arg Ser Val Ile Ala Ile Val Thr His Gly Ile Leu Ser
                245                 250                 255

Gly Lys Ala Ile Glu Asn Ile Asn Asn Ser Lys Leu Asp Arg Val Val
            260                 265                 270

Cys Thr Asn Thr Val Pro Phe Glu Glu Lys Met Lys Leu Cys Pro Lys
        275                 280                 285

Leu Asp Val Ile Asp Ile Ser Ala Val Leu Ala Glu Ser Ile Arg Arg
    290                 295                 300

Leu His Asn Gly Glu Ser Ile Ser Tyr Leu Phe Lys Asn Asn Pro Leu
305                 310                 315                 320